Europäisches Patentamt

European Patent Office

Office européen des brevets

(19)

(11) **EP 1 586 347 A1**

(12) **EUROPEAN PATENT APPLICATION**

(43) Date of publication:
**19.10.2005 Bulletin 2005/42**

(51) Int Cl.$^7$: **A61N 1/30**

(21) Application number: **05252156.4**

(22) Date of filing: **06.04.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **07.04.2004 US 821151**

(71) Applicant: **Vyteris, Inc.**
**Fair Lawn, NJ 07410 (US)**

(72) Inventors:
• **Keusch, Preston**
**Jonesboro, ME 04648 (US)**

• **Reddy, Vilambi NRK**
**Trichy 620002 Tamil Nadu (IN)**
• **Green, Philip**
**Weehawken, NJ 07088 (US)**
• **Jain, Uday**
**Plainsboro, NJ 08536 (US)**

(74) Representative: **Samuels, Lucy Alice**
**Gill Jennings & Every**
**Broadgate House**
**7 Eldon Street**
**London EC2M 7LH (GB)**

(54) **Electrically assisted lidocaine and epinephrine delivery device having extended shelf-stability**

(57) Highly shelf-stable electrically assisted transdermal drug delivery systems for deliverir g epinephrine, typically with an anesthetic such as lidocaine, are provided along with methods for making the highly shelf-stable epinephrine-containing transdermal deliver device. Highly shelf-stable packaged electrode assemblies for transdermal delivery of epinephrine also are provided.

FIG. 2

EP 1 586 347 A1

**Description**

**BACKGROUND**

**Field of the Invention**

Highly shelf-stable electrically assisted transdermal drug delivery systems for delivering epinephrine, typically with an anesthetic such as lidocaine, are provided along with methods for making the highly shelf-stable epinephrine-containing transdermal delivery device.

**Description of the Related Art**

Transdermal drug delivery systems have, in recent years, become an increasingly important means of administering drugs. Such systems offer advantages clearly not achievable by other modes of administration such as introduction of the drug through the gastro-intestinal tract or punctures in the skin, to name a few.

There are two types of transdermal drug delivery systems, "passive" and "active." Passive systems deliver drug through the skin of the user unaided, an example of which would involve the application of a topical anesthetic to provide localized relief, as disclosed in U.S. Patent No. 3,814,095. Active systems, on the other hand, use external force to facilitate delivery of a drug through a patient's skin. Examples of active systems include ultrasound, electroporation and/or iontophoresis.

Iontophoretic delivery of a medicament is accomplished by application of a voltage to a medicament-loaded reservoir-electrode, sufficient to maintain a current between the medicament-loaded reservoir-electrode and a return reservoir electrode (another electrode) applied to a patient's skin so that the desired medicament is delivered to the patient in ionic form.

Conventional iontophoretic devices, such as those described in U.S. Patent Nos. 4,820,263, 4,927,408, and 5,084,008, the disclosures of which are hereby incorporated by reference, deliver a drug transdermally by iontophoresis. These devices basically consist of two electrodes - an anode and a cathode. In a typical iontophoretic device, electric current is driven from an external power supply. In a device for delivering drug from an anode, positively charged drug is delivered into the skin at the anode, with the cathode completing the electrical circuit. Likewise, in a system for delivering drug from a cathode, negatively charged drug is delivered into the skin at the cathode, with the anode completing the electrical circuit Accordingly, there has been considerable interest in iontophoresis to perform delivery of drugs for a variety of purposes. One example is the delivery of lidocaine, a common topical, local anesthetic.

Shelf storage stability problems for many of the iontophoresis devices reported in the literature require that the medicament be stored separately from the reservoir-electrode until immediately prior to use. Iontophoretic delivery is recognized as desirable for many medicaments, but it is not widely used because, in many cases, no devices are commercially available that meet all of the needs of the potential user population. An important requirement for a product to enjoy widespread usage is shelf storage stability. If a drug product is not stable under normal distribution and shelf storage conditions, it is unlikely to be a successfully commercialized product because most or all of the product's useful life is exhausted during the time required for product manufacturing and distribution. For this reason, shelf storage or stability is an important part of a drug product's regulatory approval process - if there are difficulties with storage stability, regulatory approval may be withheld.

It has proven difficult to store drug to be delivered in a complex, multi-component reservoir-electrode. In some cases, the reservoir-electrode is maintained in a dry (unhydrated) condition prior to use, due to the tendency of the active electrode material to undergo physical and chemical changes during shelf storage in an aqueous medium. Thus, the need to store the several components separately has limited the use of iontophoretic devices, because in order to use the device, the reservoir-electrode needs to be charged with the medicament and hydrated immediately prior to use. There are regulatory requirements related to the accuracy and precision of content of a particular drug in an individual dosage form. When a drug dosage form is a tablet, there are specific requirements related to weight variation, dissolution, content and stability. Parenteral dosage forms require concentration and stability assays. Other more complex dosage forms, such as transdermal or iontophoretic delivery devices, are developing similar standards, but problems related to loading the devices and the stability of the charged devices are continuing.

Several United States Patents disclose devices that attempt to overcome the problem of shelf storage stability and facilitate the preparation of iontophoretic devices. U.S. Patent No. 5,320,598 discloses a dry-state iontophoretic drug delivery device that has drug and electrolyte reservoirs that are initially in a non-hydrated condition.

The device has a liquid-containing pouch or breakable capsules that contain water or other liquid, the liquid being releasable by disrupting the liquid containers prior to use. Commercial manufacture of such a device would be complex.

U.S. Patent No. 5,385,543 also discloses a dry-state iontophoretic drug delivery device that has drug and electrolyte reservoirs. The disclosed device includes a backing layer with at least one passageway therethrough that allows the introduction of water or other liquids into the drug and electrolyte reservoirs prior to prior to use, followed by joining the reservoirs to the electrodes. The patent teaches that by joining the reservoirs to the electrodes after hydration, delamination problems are reduced.

A different approach to the shelf storage stability problem is disclosed in U.S. Patent No. 5,817,044. In that patent, the device is divided, or otherwise separated, into at least two portions, with one portion containing the electrode reservoir and the other containing the drug reservoir, which may include, a medication in a dry form. The user causes the two portions to come into electrical-conducting contact with one another to at least partially hydrate one of the reservoirs, by either folding the device to bring the two portions into contact with one another or by removing a barrier dividing the two portions. While this device seems to be somewhat easier to use than the devices disclosed in the above patents, there currently is no such commercial device.

International Patent Publication WO 98/208869 discloses an iontophoretic device for delivery of epinephrine HCl and lidocaine HCl. The disclosed device includes materials that deter microbial growth and anti-oxidants to enhance the stability of epinephrine. While that disclosure recognizes the need for shelf storage stability and addresses the problem of epinephrine stability by including anti-oxidants, there is no teaching of: the benefits of uniformly loading the reservoir-electrode, the problem of the corrosion of the electrode in manufacture and storage and solutions thereof; reservoir contact with suitable adhesives, protective release covers, packaging materials or packaging environments; or the effect of drug on the electrode. Again, there is no commercial product based on the information in that disclosure.

A further problem related to production or a successful pharmaceutical product is related to the requirements for accuracy and precision of dosage. In some of the iontophoretic drug delivery devices described above, the user or the practitioner is required to perform some action to hydrate the reservoir-electrode and introduce the medicament to be delivered into the delivery device prior to use. Such operations that depend upon the practitioner or user to charge the medicament into the device under relatively uncontrolled conditions may result in improper dosing. Regulatory requirements for pharmaceutical products generally specify that not only medicaments contain between ninety and one hundred-ten percent of the label claim, but also that the delivery be uniform from sample to sample. It is well recognized that many medicaments are not stable under conditions necessary for assembly and storage of iontophoretic reservoir-electrodes. A method of accurately and repeatedly loading the medicament and any required stability enhancing excipients during the assembly process of reservoirs useful for passive transdermal drug delivery and reservoir-electrodes for iontophoretic drug delivery devices, that is compatible with a mechanized assembly process and also provides a drug charged reservoir-electrode with satisfactory stability properties is described in International Patent Publication No. WO 01/91848, corresponding to U.S. Patent Application No. 09/584.453, both of which are incorporated herein by reference in their entirety.

Powers *et al.*, U.S. Patent No. 4,786,277; Linkwitz *et al.*, U.S. Patent No. 6,295,469; and EP 0941 085 B1 disclose iontophoresis devices for delivery of lidocaine. Linkwitz *et al.* discloses delivery of lidocaine with epinephrine. However, the device of Linkwitz *et al.* fails to provide sufficient stability for extended shelf life. The device of Linkwitz *et al.* is shown to be stable only for about ten months, and then only in a drug-loaded hydrogel reservoir. The stability of a complete, marketable electrode assembly including an electrode was not analyzed, nor would the less than ten month stability of the hydrogel of Linkwitz *et al.* be satisfactory for commercial distribution without the difficulty of refrigeration.

Adrenaline, the natural form of epinephrine, was isolated in 1900. It was introduced into medical use in 1901. Epinephrine and its salts have had recognized stability problems since isolation. Epinephrine in free base form or as an ionic salt is labile in the presence of oxygen and the degradation is accelerated in the presence of light and salts of metal ions such as Al, Cu and Fe. Epinephrine usually is used in aqueous form alone or in combination with other drugs such as lidocaine. Epinephrine typically is stored in gas-tight containers under an inert gas such as nitrogen. The container usually limits direct light to penetrate the liquid or is stored in a secondary opaque package. Solutions containing soluble epinephrine are so unstable that even when packaged in a vial for multiple injections, they are labeled with a warning that the opened vial is not to be used after one week after its first use. Glass ampules containing an aqueous solution of epinephrine under an inert atmosphere have limited shelf lives that do not exceed 24 months. This easily can lead to compliance problems in the field when the time of first use often is ignored or not noticed. This has relevance to iontophoretic products previously and currently marketed, such as Iomed's Numby® 900 for local delivery of lidocaine and epinephrine by iontophoresis. That device is marketed as a kit containing active and return electrode pairs and a controller. A multiple-use vial of lidocaine epinephrine solution, Iontocaine™ must be purchased separately. The system has to be assembled and the liquid containing lidocaine and epinephrine is then added to the active patch just before use. It is easy for a practitioner to lose track of the age of the multi-use vial of lidocaine and epinephrine, consequently allowing the epinephrine to degrade in the vial. It also is cumbersome to preload a patch just before use. A syringe is needed for each use and the potential for dose-to-dose variation is present. For example, the loading syringe may not be filled with the proper amount of solution, some of the solution may not be applied to the patch and/or the liquid can squeeze out of the absorbent drug containing electrode because the solution is a separate phase from the absorbent reservoir, which can compromise the peripheral adhesive and compromise the efficacy of the device.

Stability of a commercially acceptable iontophoretic system for delivery of lidocaine and epinephrine involves considerations well beyond drug stability as compared to storing an aqueous lidocaine/epinephrine anesthetic solution packaged in glass vials or even in a pre-filled syringe. To date, there are no teachings on how to make a shelf-stable donor reservoir-electrode for delivery of lidocaine and epinephrine that contains the drug pre-loaded into a delivery reservoir. Besides dealing with the oxygen content of the hydrogel reservoir, the epinephrine/lidocaine-containing reservoir is in contact with a metal electrode and other parts of this drug device, such as the adhesive, nonwoven transfer pad and release cover. The fact that the silver/silver chloride typically used to prepare electrodes for iontophoretic devices typically contains trace amounts of epinephrine-degrading metals, such as copper, speaks against storage of an epinephrine-containing solution in contact with silver/silver chloride electrodes. Prior art actually teaches away from the use of epinephrine and suggests other vasoconstrictors (for example, see U.S. Patent No. 5,334,138, column 6, lines 22-38).

Teachings in the field of iontophoresis of epinephrine/lidocaine HCl products only show 13 weeks to about ten months of stability. These products show stability only for the drug-containing reservoir alone, not coupled with other device components, such as the required electrode.

In addition, conventional iontophoretic devices are not equipped with various structural, physical, mechanical, electrical and/or electromechanical features that could maximize the efficiency and effectiveness of delivery of a composition to a membrane. What are needed are improved features that can enhance the performance of such devices.

## SUMMARY

Provided is a shelf-storage stable iontophoretic device for delivery of epinephrine along with a topical anesthetic, such as lidocaine. In the device, the drug is stored as a solid solution in a solid solution reservoir thereby avoiding squeezing out of drug and changes in the active area of the reservoir. The device includes an electrode and a hydrophilic polymeric reservoir situated in electrically conductive relation to the electrode and is ready for use immediately upon removal from its packaging - there is no need to load the active ingredients in the anode reservoir or return solution in the cathode reservoir prior to use. The device is electrically stable, physically stable, electrochemically stable, microbiologically stable and chemically stable for more than 24 months at room-temperature, with stability for extended periods at elevated temperatures, making manufacture, distribution and storage more effective and providing the end user a greater confidence in the product, with less returns of the device from customer.

An electrode assembly for an electrically assisted drug delivery device is provided that includes a hermetically sealed anode assembly comprising a first electrode and a donor hydrogel comprising epinephrine in electrical contact with the first electrode, wherein the anode assembly is electrically stable, physically stable, electrochemically stable, microbiologically stable and chemically stable for at least 10 months at 25°C when hermetically sealed. The donor hydrogel typically contains an anesthetic, such as lidocaine. In one embodiment, the donor hydrogel contains an amount of sodium metabisulfite equal to or slightly greater than a minimal amount of sodium metabisulfite needed to scavenge substantially all oxygen in the packaged donor hydrogel. The electrode assembly typically is packaged within a hermetically sealed container in the presence of an inert gas. The electrode assembly can be in any useful form, for example, and without limitation, as an integrated assembly containing both the anode assembly and a cathode, or as a split electrode with the anode packaged separately from the cathode.

In a further embodiment, the electrode assembly includes a first electrode and a donor hydrogel comprising lidocaine and epinephrine in electrical contact with the first electrode. The electrode assembly is electrically stable, physically stable and chemically stable for at least 24 months at 25°C when the anode assembly is hermetically sealed.

In one specific embodiment, the electrode assembly is a hermetically sealed assembly. The electrode assembly includes a backing; a first silver/silver chloride electrode and a PVP donor hydrogel comprising lidocaine and epinephrine in electrical contact with the first electrode attached to the backing; a second silver/silver chloride electrode and a return hydrogel in electrical contact with the second electrode attached to the backing; an electrically conductive silver/silver chloride anode trace attached to the backing and in electrical contact with the first electrode; an electrically conductive silver/silver chloride cathode trace attached to the backing, and in electrical contact with the second electrode; and a dielectric layer coating the periphery of the anode and cathode traces. The donor hydrogel also includes an amount of sodium metabisulfite equal to or slightly greater than a minimal amount of sodium metabisulfite needed to scavenge substantially all oxygen in the packaged donor hydrogel and an amount of salt sufficient to prevent electrode corrosion during or after loading of the hydrogel reservoir. In this embodiment, the first and second electrodes and the anode and cathode traces can be deposited as silver/silver chloride-containing ink. The electrode assembly is stable for at least 10 months at 25°C when hermetically sealed.

Also provided is a packaged electrode assembly for an electrically assisted drug delivery device. The packaged assembly includes a hermetically sealed container and an electrode assembly sealed within the container. The electrode assembly includes a first electrode and a donor PVP hydrogel comprising lidocaine and epinephrine in electrical contact with a silver/silver chloride first electrode. The packaged electrode assembly is electrically stable, physically stable, electrochemically stable, microbiologically stable and chemically stable for at least 24 months at 25°C when the anode assembly is hermetically sealed. The donor PVP hydrogel may contains an amount of sodium metabisulfite equal to or slightly greater than a minimal amount of sodium metabisulfite needed to scavenge oxygen in the packaged donor PVP hydrogel for at least 24 months.

A method for preparing a shelf-stable electrode assembly for electrically assisted delivery of a local anesthetic and epinephrine to a patient also is provided. The electrode assembly comprises an unloaded hydrogel reservoir in electrical contact with a silver-silver chloride electrode. The unloaded hydrogel reservoir contains an amount of salt sufficient to prevent electrode corrosion during or after loading of the hydrogel reservoir. The method includes the steps of: loading the unloaded hydrogel reservoir with a loading solution containing lidocaine and epinephrine and packaging the assembly in a hermetically sealed container. In one embodiment of the method, prior to the loading step, the loading solution is absorbed into an absorbent pad attached to a releasable molded sheet configured to cover the hydrogel reservoir, and the releasable liner is attached to the electrode assembly with the absorbent pad contacting the hydrogel reservoir, thereby contacting the loading solution with the hydrogel.

## BRIEF DESCRIPTION OF THE DRAWINGS

Figure 1 (prior art) shows schematically an electrically assisted drug delivery system including an anode assembly, a cathode assembly and a controller/power supply.

Figure 2 shows an exploded isometric view of various aspects of an integrated electrode assembly provided in accordance with the present invention.

Figure 3 shows an exploded isometric view of various aspects of an integrated electrode assembly provided in accordance with the present invention.

Figure 4 shows an elevated view of various aspects of an integrated electrode assembly provided in accordance with the present invention.

Figure 5A includes an exploded isometric view illustrating various aspects of the interconnection of an integrated electrode assembly provided in accordance with the present invention with components of an electrically assisted delivery device.

Figure 5B shows a schematic representation of the interaction between a portion of an integrated electrode assembly provided in accordance with the present invention and components of an electrically assisted delivery device.

Figure 5C illustrates a schematic representation of the interaction between a portion of an integrated electrode assembly provided in accordance with the present invention and components of an electrically assisted delivery device

Figure 6 includes a schematic elevated view of various aspects of an integrated electrode assembly provided in accordance with the present invention.

Figures 6B and 6C show cross-sectional views illustrating aspects of the electrode assembly of Figure 6.

Figure 7 includes a schematic elevated view of various aspects of an integrated electrode assembly provided in accordance with the present invention.

Figure 7A includes a cross-sectional view of the release cover of Figure 7.

Figure 8 includes a schematic that illustrates the effect of electrode geometry and spacing on the delivery paths of a composition through a membrane.

Figure 9 includes a schematic that illustrates the effect of electrode geometry and spacing on the delivery paths of a composition through a membrane.

Figure 10 shows a cross-sectional view of a schematic un-loaded electrode assembly in contact with a loading solution.

Figure 11 is a cut-away view of a package including an electrode assembly structured in accordance with the present invention.

Figures 12-14 are linear regression plots for the lidocaine hydrochloride potency assay data at 25°C/60% RH for lots 1, 3 and 3, respectively.

Figures 15-17 are linear regression plots for the epinephrine potency assay data at 25°C/60% RH for lots 1, 2 and 3, respectively. LSL and USL refer to Lower Specification Limit and Upper Specification Limit, respectively.

Figures 18A and 18B are graphs showing accumulation in micrograms per patch of epinephrine sulfonic acid at 25°C for 24 months (Figure 18A) and at 40°C for 6 months (Figure 18B).

## DETAILED DESCRIPTION

The use of numerical values in the various ranges specified in this application, unless expressly indicated otherwise, are stated as approximations as though the minimum and maximum values within the stated ranges were both preceded by the word "about." In this manner, slight variations above and below the stated ranges can be used to achieve substantially the same results as values within the ranges. Also, the disclosure of these ranges is intended as a continuous range including every value between the minimum and maximum values.

Unless otherwise specified, embodiments of the present invention are employed under "normal use" conditions, which refer to use within standard operating parameters for those embodiments. During operation of various embodiments described herein, a failure rate of one or more parameters of about 10% or less for an iontophoretic device under "normal use" is considered an adequate failure rate for purposes of the present invention.

Described herein is an electrode assembly for electrically assisted transmembrane delivery of drugs, for example lidocaine and epinephrine. The electrode assembly exhibits exceptional shelf-stability, even at temperatures greater than room temperature (25°C).

The terms "unloaded" or "unloaded reservoir," are necessarily defined by the process of loading a reservoir. In the loading process, a drug or other compound or composition if absorbed, adsorbed and/or diffused into a reservoir to reach a final content or concentration of the compound or composition. An unloaded reservoir is a reservoir that lacks that compound or composition in its final content or concentration. In one example, the unloaded drug reservoir is a hydrogel, as described in further detail below, that includes water and a salt. One or more additional ingredients may be included in the unloaded reservoir. Typically, active ingredients are not present in the unloaded gel reservoir. Other additional, typically non-ionic ingredients, such as preservatives, may be included in the unloaded reservoir. Although the salt may be one of many salts, including alkaline metal halide salts, the salt typically is sodium chloride. Other halide salts such as, without limitation, KCl or LiCl might be equal to NaCl in terms of functionality, but may not be preferred. Use of halide salts to prevent electrode corrosion is disclosed in U.S. Patent Nos. 6,629,968 and 6,635,045 both of which are incorporated herein by reference in their entireties.

The term "electrically assisted delivery" refers to the facilitation of the transfer of any compound across a membrane, such as, without limitation, skin, mucous membranes and nails, by the application of an electric potential across that membrane. "Electrically assisted delivery" is intended to include, without limitation, iontophoretic, electrophoretic and electroendosmotic delivery methods. By "active ingredient," it is meant, without limitation, drugs, active agents, therapeutic compounds and any other compound capable of eliciting any pharmacological effect in the recipient that is capable of transfer by electrically assisted delivery methods. A "transdermal device" or "transdermal patch" includes both active and passive transdermal devices or patches.

The term "lidocaine", unless otherwise specified, refers to any water-soluble form of lidocaine, including salts or derivatives, homologs or analogs thereof For example, as is used in the Examples below, "lidocaine" refers to lidocaine hydrochloride (HCl), commercially available as XYLOCAINE, among other names.

The term "epinephrine" refers to any form of epinephrine, salts, its free base or derivatives, homologs or analogs thereof so long as they can be solubilized in an aqueous solution. For example, as is used in the examples below, "epinephrine" refers to epinephrine bitartrate.

As applied to various embodiments of electrically assisted delivery devices described herein, the term "integrated" as used in connection with a device indicates that at least two electrodes are associated with a common structural element of the device. For example, and without limitation, a transdermal patch of an iontophoretic device may include both a cathode and an anode "integrated" therein, i.e., the cathode and anode are attached to a common backing.

As applied to various embodiments of electrically assisted delivery devices described herein, a "flexible" material or structural component is generally compliant and conformable to a variety of membrane surface area configurations and a "stiff" material or structural component is generally not compliant and not conformable to a variety of membrane surface area configurations. In addition, a "flexible" material or component possesses a lower flexural rigidity in comparison to a "stiff" material or structural component having a higher flexural rigidity. For example and without limitation, a flexible material when used as a backing for an integrated patch can substantially conform over the shape of a patient's forearm or inside elbow, whereas a comparatively "stiff" material would not substantially conform in the same use as a backing.

As applied herein, the term "transfer absorbent" includes any media structured to retain therein a fluid or fluids on an at least temporary basis and to release the retained fluids to another medium such as a hydrogel reservoir, for example. Examples of "transfer absorbents" that may be employed herein include, without limitation, non-woven fabrics and open-cell sponges.

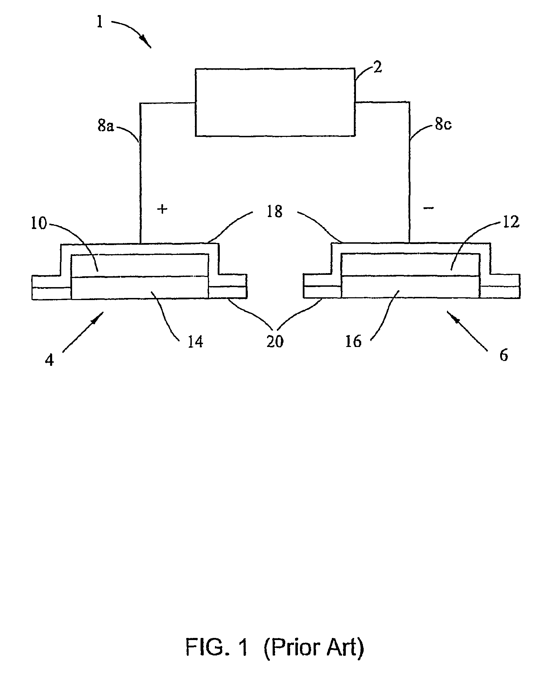

Figure 1 depicts schematically a typical electrically assisted drug delivery apparatus 1. The apparatus 1 includes an electrical power supply/controller 2, an anode electrode assembly 4 and a cathode electrode assembly 6. Anode electrode assembly 4 and cathode electrode assembly 6 are connected electrically to the power supply/controller 2 by conductive leads 8a and 8c (respectively). The anode electrode assembly 4 includes an anode 10 and the cathode electrode assembly 6 includes a cathode 12. The anode 10 and the cathode 12 are both in electrical contact with the leads 8a, 8c. The anode electrode assembly 4 further includes an anode reservoir 14, while the cathode electrode assembly 6 further includes a cathode reservoir 16. Both the anode electrode assembly 4 and the cathode electrode assembly 6 include a backing 18 to which a pressure sensitive adhesive 20 is applied in order to affix the electrode assemblies 4, 6 to a membrane (e.g., skin of a patient), to establish electrical contact for the reservoirs 14, 16 with the membrane. Optionally, the reservoirs 14, 16 may be at least partially covered with the pressure sensitive adhesive 20.

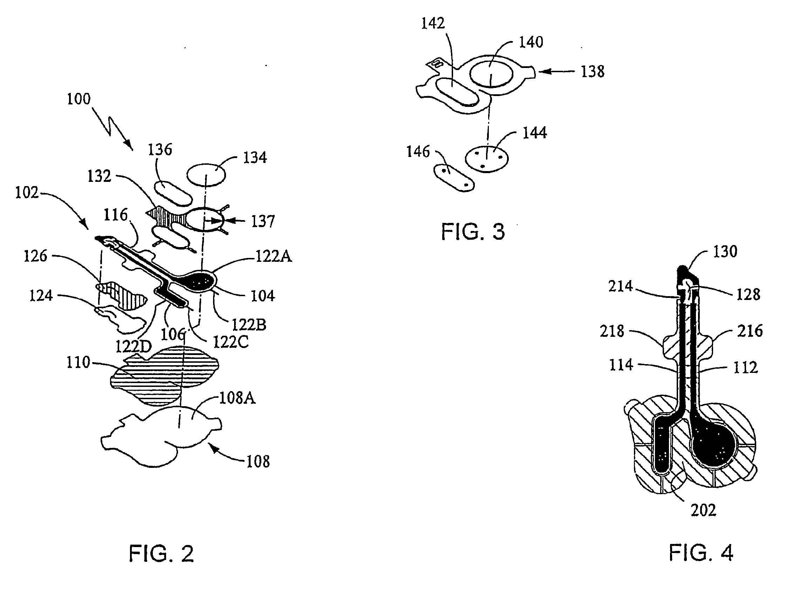

Figures 2 through 10 illustrate various aspects of an integrated electrode assembly 100 of the present invention structured for use with an electrically assisted delivery device, for example, for delivery of a composition to a membrane. A printed electrode layer 102 including two electrodes (an anode 104 and a cathode 106) is connected to a flexible backing 108 by a layer of flexible backing adhesive 110 positioned between the printed electrode layer 102 and the flexible backing 108. One or more leads 112, 114 may extend from the anode 104 and/or cathode 106 to a tab end portion 116 of the printed electrode layer 102. In various aspects, an insulating dielectric coating 118 may be deposited on and/or adjacent to at least a portion of one or more of the electrodes 104, 106 and/or the leads 112,114. The dielectric coating 118 may serve to strengthen or bolster the physical integrity of the printed electrode layer 102; to reduce point source concentrations of current passing through the leads 112, 114 and/or the electrodes 104, 106; and/or to resist creating an undesired short circuit path between portions of the anode 104 and its associated lead 112 and portions of the cathode 106 and its associated lead 114.

In other aspects, one or more splines 122A, 122B, 122C, 122D may be formed to extend from various portions of the printed electrode layer 102, as shown. It can be seen that at least one advantage of the splines 122 is to facilitate manufacturability (e.g., die-cutting of the electrode layer 102) and construction of the printed electrode layer 102 for use in the assembly 100. The splines 122 may also help to resist undesired vacuum formation when a release cover (see discussion hereafter) is positioned in connection with construction or use of the assembly 100.

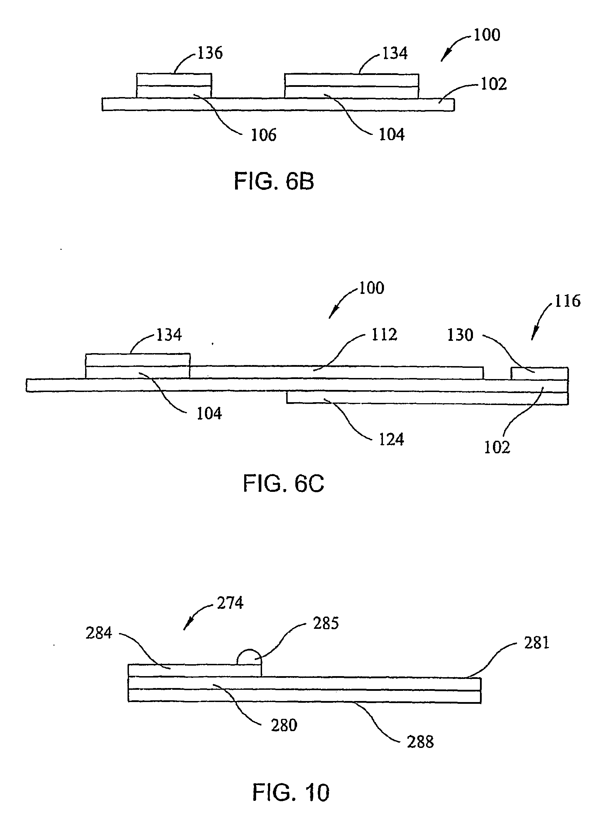

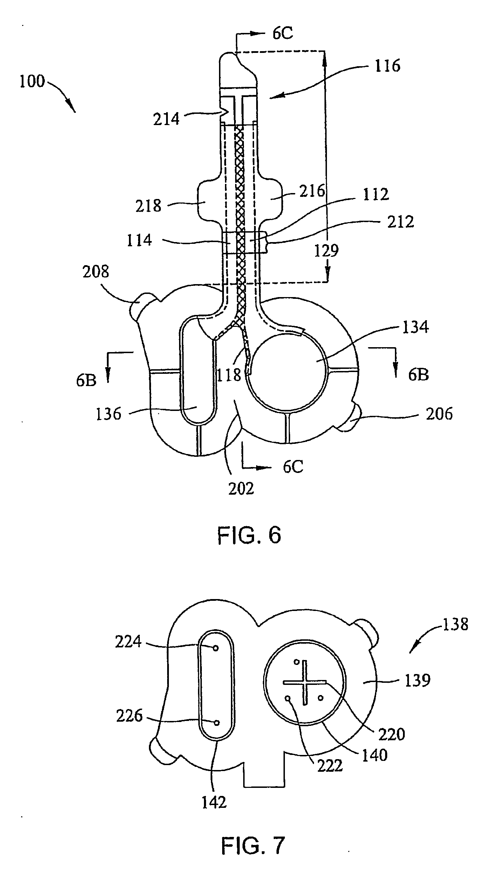

In other embodiments of the present invention, a tab stiffener 124 is connected to the tab end portion 116 of the printed electrode layer 102 by a layer of adhesive 126 positioned between the tab stiffener 124 and the tab end portion 116. In various embodiments, a tab slit 128 may be formed in the tab end portion 116 of the assembly 100 (as shown more particularly in Figures 2 and 4). The tab slit 128 may be formed to extend through the tab stiffener 124 and the layer of adhesive 126. In other embodiments, a minimum tab length 129 (as shown particularly in Figure 6) as structured in association with the tab end portion 116 may be in the range of at least about 1-5 inches.

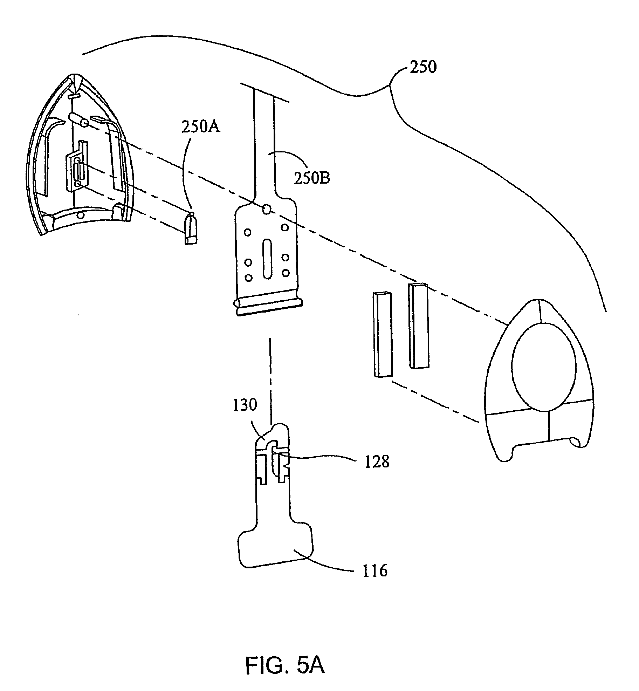

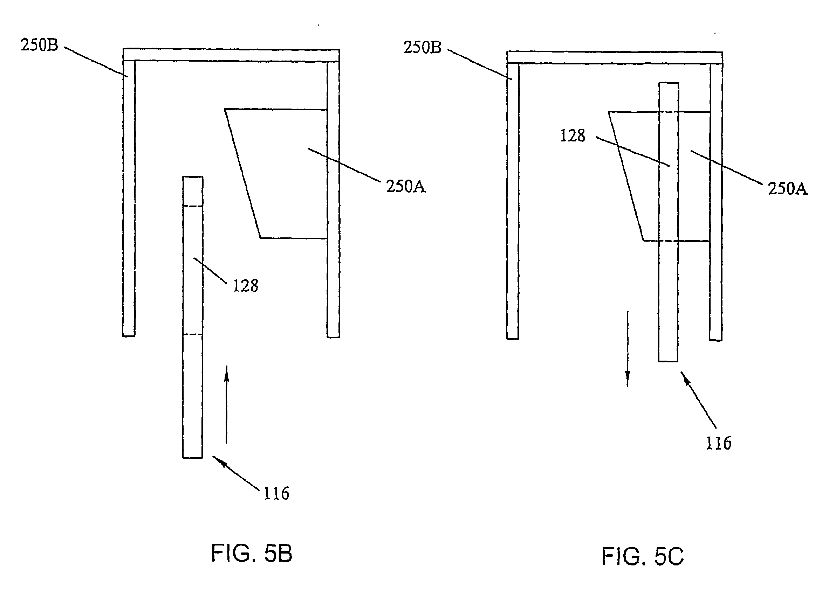

With reference to Figures 5A - 5C, the tab end portion 116 may be structured to be mechanically or electrically operatively associated with one or more components of an electrically assisted drug delivery device such as a knife edge 250A of a connector assembly 250, for example. As shown schematically in Figures 5B and 5C, once the tab end portion 116 is inserted into a flexible circuit connector 250B of the connector assembly 250, the tab slit 128 of the tab end portion 116 may be structured to receive therein the knife edge 250A. It can be appreciated that the interaction between the knife edge 250A and the tab slit 128 may serve as a tactile sensation aid for a user manually inserting the tab end portion 116 into the flexible circuit connector 250B of the connector assembly 250. In addition, the knife edge 250A may be structured, upon removal of the tab end portion 116 from the connector assembly 250, to cut or otherwise disable one or more electrical contact portions positioned on the tab end portion 116, such as a sensor trace 130, for example. It can be seen that this disablement of the electrical contact portions may reduce the likelihood that unintended future uses of the assembly 100 will occur after an initial use of the assembly 100 and the connector assembly 250 for delivery of a composition to a membrane, for example.

In other aspects, a layer of transfer adhesive 132 may be positioned in communication with the printed electrode layer 102 to facilitate adherence and/or removal of the assembly 100 from a membrane, for example, during operation of an electrically assisted delivery device that includes the assembly 100. As shown in Figure 2, a first hydrogel reservoir 134 is positioned for communication with the anode 104 of the printed electrode layer 102 and a second hydrogel reservoir 136 is positioned for communication with the cathode 106 of the printed electrode layer 102. In other aspects, although a hydrogel may be preferred in many instances, there may be substantially no hydrogel reservoir associated with the cathode 106, or a substance including NaCl, for example, maybe associated with the cathode 106.

As shown in Figure 3, a release cover 138 includes an anode-donor portion 140 and a cathode-return portion 142. The anode-donor portion 140 is structured to receive therein a donor transfer absorbent 144 suitably configured/sized for placement within the anode-donor portion 140. Likewise, the cathode-return portion 142 is structured to receive therein a return transfer absorbent 146 suitably configured/sized for placement within the cathode-return portion 142. The transfer absorbents 144, 146 may be attached to their respective portions 140, 142 by a suitable method or apparatus, such as by use of one or more spot welds, for example. In construction of the assembly 100, it can be seen that the release cover 138 is structured for communication with the flexible backing adhesive layer 110 such that the donor transfer absorbent 144 establishes contact with the hydrogel reservoir 134 associated with the anode 104 and the return transfer absorbent 146 establishes contact with the hydrogel reservoir 136 associated with the cathode 106.

In various embodiments, the integrated assembly 100 may include a first reservoir-electrode assembly (including the reservoir 134 and the anode 104) charged with lidocaine HCl and epinephrine bitartrate, for example, that may function as a donor assembly and a second reservoir-electrode assembly (including the reservoir I36 and the cathode 106) that may function as a return assembly. The assembly 100 includes the reservoir-lectrode 104 and the reservoir-electrode 106 mounted on an electrode assembly securement portion 108A of the flexible backing 108. The assembly 100 includes two electrodes, an anode 104 and a cathode 106, each having an electrode surface and an operatively associated electrode trace or lead 112 and 114, respectively. The electrodes 104, 106 and the electrode traces 112, 114 may be formed as a thin film deposited onto the electrode layer 102 by use of a conductive ink, for example. The conductive ink may include Ag and Ag/AgCl, for example, in a suitable binder material, and the conductive ink may have the same composition for both the electrodes 104, 106 and the electrode traces 112, 114. A substrate thickness for the conductive ink may be in the range of about 0.002 inches to 0.007 inches. In other aspects, the specific capacity of the conductive ink is preferably in the range of about 2 to 120 mA-min/cm$^2$, or more preferably in the range of 5 to 20 mA-min /cm$^2$. In various aspects, the conductive ink may comprise a printed conductive ink. The electrodes 104, 106 and the electrode traces 112, 114 may be formed in the electrode layer 102 to comprise a stiff portion of the assembly 100.

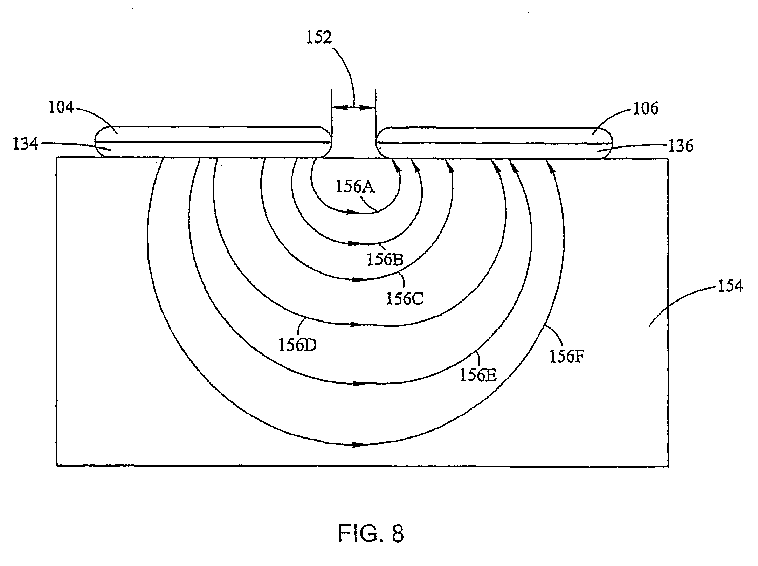

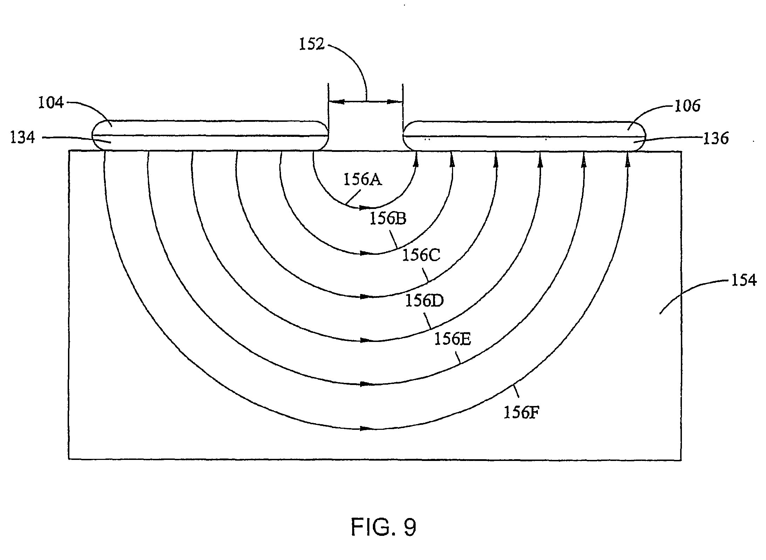

In various embodiments of the present invention, a shortest distance 152 between a surface area of the anode 104 / reservoir 134 assembly and a surface area of the cathode 106 / reservoir 136 assembly may be in the range of at least about 0.25 inches. Referring now to Figure 8, for example, it can be seen that inappropriate selection of the distance 152, the geometric configuration of the electrodes 104, 106 (e.g., thickness, width, total surface area, and others), and/or a combination of other factors may result in a substantially non-uniform delivery of a composition between the electrodes through a membrane 154 during operation of the assembly 100. As shown, the delivery of the composition through the membrane is shown schematically by composition delivery paths 156A - 156F. In contrast, as shown in Figure 9, appropriate selection of the distance 152, the geometric configuration of the electrodes 104, 106 (e.g., thickness, width, total surface area, and others), and/or a combination of other factors may result in a substantially uniform delivery of a composition between the electrodes through a membrane 154 as shown by delivery paths 156A - 156F. It can be seen that the inventors have recognized the problem of delivering a composition through a membrane that may include scar tissue, for example, or another variation in the density of the membrane that may adversely impact the effectiveness and uniformity of delivery of the composition between the electrodes of a device, for example.

In accordance with discussion above, the electrodes 104, 106 may each be mounted with bibulous reservoirs 134, 136 (respectively) formed from a cross-linked polymeric material such as cross-linked poly(vinylpytrolidone) hydrogel, for example, including a substantially uniform concentration of a salt, for example. The reservoirs 134, 136 may also include one or more reinforcements, such as a low basis weight non-woven scrim, for example, to provide shape retention to the hydrogels. The reservoirs 134, 136 each may have adhesive and cohesive properties that provide for releasable adherence to an applied area of a membrane (e.g., the skin of a patient). In various embodiments, the strength of an adhesive bond formed between portions of the assembly 100 and the application area or areas of the membrane is less than the strength of an adhesive bond formed between the membrane and the reservoirs 134, 136. These adhesive and cohesive properties of the reservoirs 134, 136 have the effect that when the assembly 100 is removed from an applied area of a membrane, a substantial amount of adhesive residue, for example, does not remain on the membrane. These properties also permit the reservoirs 134, 136 to remain substantially in communication with their respective electrodes 104, 136 and the flexible backing 108 to remain substantially in communication with the printed electrode layer 102.

Portions of the assembly 100, as provided in accordance with embodiments of the present invention, may be structured to exhibit flexibility or low flexural rigidity in multiple directions along the structure of the device 100. Working against flexibility of the device 100, however, may be the construction of the comparatively stiffer electrode layer 102, which may include a material such as print-treated PET, for example, as a substrate. PET is a relatively strong material exhibiting high tensile strength in both the machine and transverse directions and having a flexural rigidity, $G=E\delta^n$, which is a function of modulus of elasticity (E) and a power of the thickness ($\delta$) of the material. By way of a hypothetical counter-example, if a substance such as Mylar, for example, were to be used for both the electrode layer 102 and the flexible backing 108, at least two problems would be presented: (1) the assembly 100 would be too inflexible to fully or effectively adhere to a site of treatment on a membrane, and (2) upon removal from the membrane once treatment is completed, the assembly 100 would require a relatively high level of force, due to the strength of the flexible backing 108, to remove the assembly 100.

Embodiments of the present invention provide the flexible backing 108 around the periphery of the stiff electrode layer 102. In certain aspects, a relatively thin and highly compliant flexible backing composed of about 0.004 inch EVA, for example, may be used for the flexible backing 108. This configuration offers a flexible and compliant assembly 100 in multiple planar directions, permitting the assembly 100 to conform to the contour of a variety of membranes and surfaces. In addition, a pressure sensitive adhesive (e.g., PIB) may be applied as the transfer adhesive layer 132 to mitigate a potential decrease in flexibility of the flexible backing 108. It can be seen that, in various embodiments, devices constructed in accordance with the present invention permit a degree of motion and flexure during treatment without disrupting the function of the assembly 100. The assembly 100 therefore exhibits low flexural rigidity in multiple directions, permitting conformability of the assembly 100 to a variety of membrane surface area configurations in a manner that is substantially independent of the chosen orientation of the assembly 100 during normal use. In various embodiments, a flexural rigidity of at least a portion of the flexible backing 108 is less than a flexural rigidity of at least a portion of the electrode layer 102.

In general, one advantage of the embodiments of the present invention is realized in minimization of the "footprint" of the assembly 100 when the assembly 100 is applied to a membrane to deliver a composition. As applied herein, the term "footprint" refers to the portion or portions of the assembly 100 that contact a membrane surface area (e.g., a patient's skin) during operation of the assembly 100. In certain aspects, the surface area of an assembly including the donor electrode 104 and the donor reservoir 134 may be structured to be greater than the surface area of an assembly including the return electrode 106 and the return reservoir 134 to limit the effect of the return assembly on the overall footprint of the assembly 100. In addition, the length of the distance 152 that provides separation between the anode 104 and cathode 106 may also impact the footprint. Furthermore, the size of the electrodes 104, 106 relative to their respective reservoirs 134, 136 may also affect the footprint of the assembly 100. In certain aspects, the reservoirs 134, 136 should be at least substantially the same size as their respective electrodes 104, 106.

It can be appreciated that the inventors have also recognized that once the surface area of the electrode layer 102 is fixed, including configuration of the anode 104 and cathode 106 separation distance 152, the assembly 100 should be sufficiently flexible and adherent for use on a membrane (e.g., a patient's skin). These objectives may depend on the peripheral area of the transfer adhesive layer 132 that smrounds the stiff electrode layer 102. In various embodiments, the width of the peripheral area of the transfer adhesive layer 132 adjacent to ore or both of the anode 104 and cathode 106 may be provided as a minimum width 137 (as shown, for example, in Figure 1). The minimum width 137 may be structured, in certain aspects, in the range of at least about 0.375 inches. In tum, these objectives depend on the aggressiveness of the transfer adhesive layer 132 and the flexible backing 108, which is preferably flexible and compliant as a function of the strength (e.g., modulus of elasticity) and thickness of the flexible backing 108. Any sufficiently thin material may be flexible (such as ultra-thin PET, for example), but another problem arises in that the transfer adhesive layer 132 and the flexible backing 108 should be capable of removal from a membrane with minimum discomfort to a patient, for example. Consequently, a compliant (i.e., low strength) flexible backing 108 may be employed while maintaining adequate strength for treatments using the assembly 100.

In various example aspects of the structure of the present invention, the footprint area of the assembly 100 may be preferably in the range of about 3 cm$^2$ to 100 cm$^2$, more preferably in the range of about 5 cm$^2$ to 60 cm$^2$, and most preferably in the range of about 22 cm$^2$ to 30 cm$^2$. In addition, the total electrode 104, 106 area may be in the preferred range of about 2 cm$^2$ to 50 cm$^2$ or more preferably in the range of about 4 cm$^2$ to 40 cm$^2$. In one operational example, the total contact area for the electrodes 104,106 is about 6.3 cm$^2$ and the total reservoir 134, 136 contact area is about 7.5 cm$^2$. The ratio of the area of each reservoir 134, 136 to its corresponding electrode 104, 106 may be in the range of about 1.0 to 1.5. In other aspects, the flexible backing adhesive 110 for the printed electrode layer 102 may have a thickness in the range of about 0.0015 inches to about 0.005 inches. The flexible backing 108 may be comprised of a suitable material such as EVA, polyolefins, PE, PU, and/or other similarly suitable materials.

In other example aspects of the structure of the present invention, the ratio of total electrode surface area to total footprint area may be in the range about 0.1 to 0.7, or preferably about 0.24. In certain aspects, the ratio of donor electrode 104 surface area to return electrode 106 surface area m ay be in the range of about 0.1 to 5.0, or preferably about 1.7. In still other aspects, the ratio of donor reservoir 134 thickness to return reservoir 136 thickness may be in the range of about 0.5 to 2.0, or more preferably about 1.0.

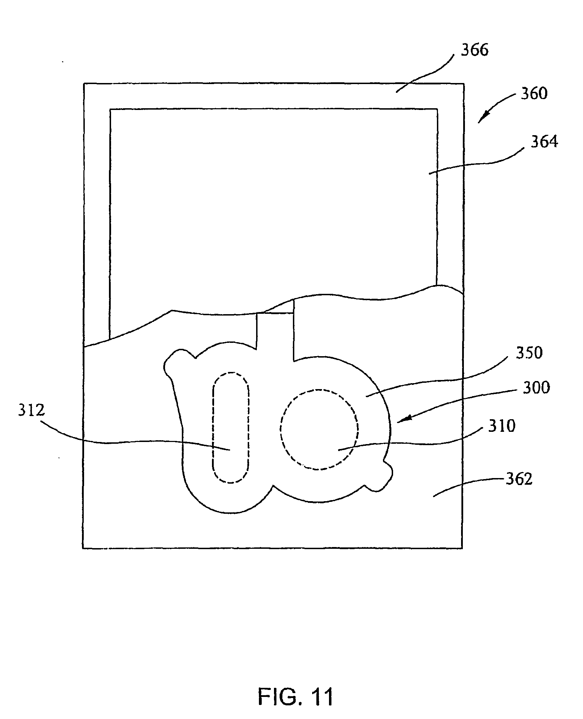

In various embodiments, the donor electrode reservoir 134, for example, may be loaded with an active ingredient from an electrode reservoir loading solution by placing an aliquot of the loading solution directly onto the hydrogel reservoir and permitting the loading solution to absorb and diffuse into the hydrogel over a period of time. Figure 10 illustrates this method for loading of electrode reservoirs in which an aliquot of loading solution is placed on the hydrogel reservoir for absorption and diffusion into the reservoir. Figure 10 is a schematic cross-sectional drawing of an anode electrode assembly 274 including an anode 280 and an anode trace 281 on a backing 288 and an anode reservoir 284 in contact with the anode 280. An aliquot of a loading solution 285, containing a composition to be loaded into the reservoir 284 is placed in contact with reservoir 284. Loading solution 285 is contacted with the reservoir 284 for a time period sufficient to permit a desired amount of the ingredients in loading solution 285 to absorb and diffuse into the gel reservoir 284. It can be appreciated that any suitable method or apparatus known to those in the art may be employed for loading the reservoir 284 with a composition.

In other embodiments of the present invention, at least one of the hydrogel reservoirs 134, 136 is positioned for communication with at least a portion of at least one of the electrodes 104, 106. In various aspects, a surface area of at least one of the hydrogel reservoirs 134, 136 may be greater than or equal to a surface area of its corresponding electrode 104, 106. At least one of the hydrogel reservoirs 134, 136 may be loaded with a composition to provide a loaded hydrogel reservoir below an absorption saturation of the loaded hydrogel reservoir. In addition, at least one component of the assembly 100 in communication with, or in the vicinity of, the loaded hydrogel reservoir may have an aqueous absorption capacity less than an aqueous absorption capacity of the loaded hydrogel reservoir. In certain embodiments, a first kind of material comprising the unloaded hydrogel reservoir 134 in communication with the anode electrode 104 is substantially identical to a second kind of material comprising the second unloaded hydrogel reservoir 136 in communication with the cathode electrode 106.

In other embodiments of the present invention, a slit 202 may be formed in the flexible backing 108 in an area located between the anode 104 and the cathode 106 of the assembly 100. The slit 202 facilitates conformability of the assembly 100 to a membrane by dividing stress forces between the portion of the assembly including the anode and the portion of the assembly including the cathodes. In various embodiments, the electrode assembly 100 includes one or more non-adhesive tabs 206 and 208 that extend from the flexible backing 108 and to which no type of adhesive is applied. The non-adhesive tabs 206, 208 permit, for example, ready separation of the release cover 138 from its attachment to the electrode assembly 100. The non-adhesive tabs 206, 208 also may facilitate removal of the assembly 100 from a membrane (e.g., a patient's skin) on which the assembly 100 is positioned for use.

As described above, at least a portion of at least one of the anode electrode trace 112 and the cathode electrode trace 114 may be covered with an insulating dielectric coating 118 at portions along the traces 112, 114. The insulating dielectric coating 118 maybe structured not to extend to cover completely the portion of the traces 112, 114 located at the tab end portion 116 of the assembly 100. This permits electrical contact between the traces 112, 114 and the electrical contacts of an interconnect device such as the flexible circuit connector 250B of the connector assembly 250. In various embodiments, the dielectric coating 118 may cover at least a portion of at least one of the anode 104 / reservoir 134 assembly and/or the cathode 106 / reservoir 136 assembly. In addition, the dielectric coating 118 may cover substantially all or at least a portion of a periphery of at least one of the electrodes 104, 106 and/or the traces 112, 114.

In various embodiments of the present invention, a gap 212 may be provided between a portion of the layer of transfer adhesive 132 nearest to the tab end portion 116 and a portion of the tab stiffener 124 nearest to the layer of transfer adhesive 132 to facilitate removal or attachment of the assembly 100 from/to a component of an electrically assisted delivery device such as the connector assembly 250, for example. In certain example embodiments, the gap 212 is at least about 0.5 inches in width. The gap 212 provides a tactile sensation aid such as for manual insertion, for example, of the assembly 100 into the flexible circuit connector 250B of the connector assembly 250. The gap 212 may also provide relief from stress caused by relative movement between the assembly 100 and other components of a delivery device (e.g., the connector assembly 250) during adhesion and use of the assembly 100 on a membrane.

In addition, at least one tactile feedback notch 214 and one or more wings 216, 218 may be formed in or extend from the tab end 116 of the electrode assembly 100. The feedback notch 214 and/or the wings 216, 218 may be considered tactile sensation aids that facilitate insertion or removal of the tab end 116 into/from a component of an electrically assisted delivery device such as, for example, to establish an operative association with the flexible circuit connector 250B of the connector assembly 250.

Figures 6B and 6C each show the layering of elements of the electrode assembly 100 as shown in Figure 6. In Figures 6B and 6C, it can be seen that the thickness of layers is not to scale and adhesive layers are omitted for purposes of illustration. Figure 6B shows a cross section of the anode electrode 104 / reservoir 134 assembly and the cathode electrode 106 / reservoir 136 assembly. The anode 104 and the cathode 106 are shown layered on the printed electrode layer 102. The anode reservoir 134 and the cathode reservoir 136 are shown layered on the anode 104 and the cathode 106, respectively. Figure 6C is a cross-sectional view through the anode 104, the anode trace 112, and the anode reservoir 134. The anode 104, the anode trace 112 and a sensor trace 130 are layered upon the electrode layer 102. The anode reservoir 134 is shown in communication with the anode 104. The tab stiffener 124, which may be composed of an acrylic material, for example, is shown attached to the tab end 116 of the assembly 100. In addition, the sensor trace 130 may be located at the tab end 116 of the electrode assembly 100.

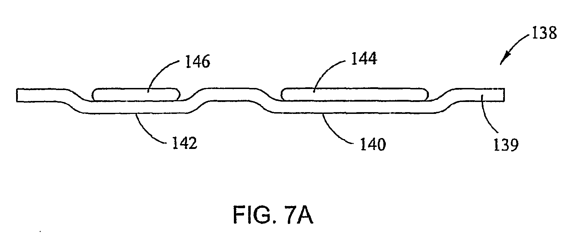

In other embodiments of the present invention. Figures 7 and 7A show schematically the release cover 138 structured for use with various devices, electrode assembles and/or systems of the present invention. The release cover 138 includes a release cover backing 139, which includes an anode absorbent well 140 and a cathode absorbent well 142. In various exemplary aspects, a nonwoven anode absorbent pad may be contained within the anode absorbent well 140 as the transfer absorbent 144, and a nonwoven cathode absorbent pad may be contained within the cathode absorbent well 142 as the transfer absorbent 146. In use, the release cover 138 is attached to the electrode assembly 100 so that the anode absorbent pad 144 and the cathode absorbent pad 146 substantially cover the anode reservoir 134 and the cathode reservoir 136, respectively. The anode absorbent pad 144 and the cathode absorbent pad 146 may each be slightly larger than their corresponding anode reservoir 134 or cathode reservoir 136 to cover and protect the reservoirs 134, 136. The anode absorbent pad 144 and the cathode absorbent pad 146 may also be slightly smaller than the anode absorbent well 140 and the cathode absorbent well 142, respectively. In various embodiments, one or more indicia 220 (e.g., a "+" symbol as shown) may be formed on at least a portion of the flexible backing 108 of the assembly 100 adjacent to the anode well 140 and/or the donor well 142. It can be appreciated that the indicia 220 may promote correct orientation and use of the assembly 100 during performance of an iontophoretic procedure, for example.

The anode absorbent pad 144 and the cathode absorbent pad 146 may be attached to the backing 139 of the release cover 138 by one or more ultrasonic spot welds such as welds 222, 224, 226, for example, as shown in Figure 7. The welds 222, 224, 226 may be substantially uniformly distributed in areas of connection between the non-woven fabric pads 144, 146 and the wells 140, 142, respectively.

To facilitate removal of the release cover 138 from the electrode assembly 100 portions of the backing 139 in communication with the transfer adhesive 132 when the release cover 138 is attached to the electrode assembly l00 may be treated with a release coating, such as a silicone coating, for example.

Figure 11 is a breakaway schematic representation of the electrode assembly 300 within a hermetically sealed packaging 360. Packaged electrode assembly 300 is shown with release liner 350 in place and anode 310 and cathode 312 are shown in phantom for reference. Hermetically sealed packaging 360 is a container that is formed from a first sheet 362 and a second sheet 364, which are sealed along seam 366. Hermetically sealed packaging 360 can be of any suitable composition and configuration, so long as, when sealed, substantially prevents permeation of any fluid or gas including, for example, permeation of oxygen into the packaging 360 and/or the loss of water from the packaging 360 after the electrode assembly 300 is sealed inside the hermetically sealed packaging 360.

In use, sheets 362 and 364 are sealed together to form a pouch after electrode assembly 300 is placed on one of sheets 362 and 364. Other techniques well-known to those skilled in the art of packaging may be used to form a hermetically sealed package with an inert atmosphere. In one embodiment, the moles of oxygen in the inert gas in the sealed pouch is limited, by controlling the oxygen concentration in the inert gas and by minimizing the internal volume, or headspace, of the package, to be slightly less than the amount of sodium metabisulfite in the epinephrine-containing reservoir needed to react with all oxygen in the package. Electrode assembly 300 is then inserted between sheets 362 and 364, an inert gas, such as nitrogen is introduced into the pouch to substantially purge air from the pouch, and the hermetically sealed packaging 360 is then sealed. The hermetically sealed packaging 360 may be sealed by adhesive, by heat lamination or by any method know to those skilled in the art of packaging devices such as electrode-assembly 300. It should be noted that sheets 362 and 364 may be formed from a single sheet of material that is folded onto itself, with one side of hermetically sealed packaging 360 being a fold in the combined sheet, rather than a seal. In other embodiments, the sheets 362, 364 may be formed from individual sheets that are laminated together, for example, to form a package. Other container configurations would be equally suited for storage of electrode-assembly, so long as the container is hermetically sealed.

Sheets 362 and 364, and in general, hermetically sealed packaging 360 may be made form a variety of materials. In one embodiment, the materials used to form hermetically sealed packaging 360 has the structure 48 gauge PET (polyethylene terephthalate)/Primer/151b LDPE (low density polyethylene)/1.0 mil aluminum foil adhesive/48 gauge PET/10 lb LDPE chevron pouch 2 mil peelable layer. Laminates of this type (foil, olefinic films and binding adhesives) form strong and channel-free seals and are essentially pinhole-free, assuring essentially zero transfer of gases and water vapor for storage periods up to and exceeding 24 months. Other suitable barrier materials to limit transport of oxygen, nitrogen and water vapor for periods of greater than 24 months are well-known to those of skill in the art, and include, without limitation, aluminum foil laminations, such as the Integra® products commercially available from Rexam Medical Packaging of Mundelein, Illinois.

It can be appreciated that any of the assemblies, devices, systems, or other apparatuses described herein may be, where structurally suitable, included within hermetically sealed packaging as described above.

In use, electrode reservoirs described herein can be loaded with an active ingredient from an electrode reservoir loading solution according to any method suitable for absorbing and diffusing ingredients into a hydrogel. Two methods for loading a hydrogel include, without limitation, placing the hydrogel in contact with an absorbent pad, material, such as a nonwoven material, into which a loading solution containing the ingredients is absorbed. A second loading method includes the step of placing an aliquot of the loading solution directly onto the hydrogel and permitting the loading solution to absorb and diffuse into the hydrogel over a period of time.

In the first protocol, the loading solution containing ingredients to be absorbed and diffused into the respective anode reservoir 134 and cathode reservoir 136 are first absorbed into the nonwoven anode absorbent pad 144 and nonwoven cathode absorbent pad 146, respectively. When a release cover thus loaded is connected to electrode assembly 100, the ingredients therein desorb and diffuse from the absorbent pads 144 and 146 and into the respective reservoir. In this case, absorption and diffusion from the reservoir cover into the reservoirs has a transfer efficiency of about 95%, requiring that about a 5% excess of loading solution be absorbed into the absorbent pads. Despite this incomplete transfer, the benefits of this loading process, as compared to placing a droplet of loading solution onto the reservoirs and waiting between about 16 and 24 hours or so for the droplet to immobilize and absorb, are great because once the release cover is laminated onto the electrode assembly, the assembly can be moved immediately for further processing and placed in inventory. There is no requirement that the assembly is kept flat and immobile while awaiting completion of absorption and/or diffusion.

The transfer absorbents 144 and 146 are typically a nonwoven material. However, other absorbents may be used, including woven fabrics, such as gauze pads, and absorbent polymeric compositions such as rigid or semi-rigid open cell foams. In the particular embodiments described herein, the efficiency of transfer of loading solution from the absorbert pads of the release cover to the reservoirs is about 95%. It would be appreciated by those skilled in the art of the present invention that this transfer efficiency will vary depending on the composition of the absorbent pads and the reservoirs as well as additional physical factors including, without limitation, the size, shape and thickness of the reservoirs and absorbent pads and the degree of compression of the absorbent pad and reservoir when the release cover is affixed to the electrode assembly. The transfer efficiency for any given release cover-electrode assembly combination can be readily determined empirically and, therefore, the amount of loading solution needed to fully load the reservoirs to their desired drug content can be readily determined to target specifications.

As discussed above, Figure 10 illustrates the second protocol for loading of electrode reservoirs in which an aliquot of loading solution is placed on the hydrogel reservoir for absorption and diffusion into the reservoir. The transfer absorbents 144, 146 typically are not included in the release cover for electrode assemblies having reservoirs loaded by this method.

In various embodiments, the electrode assembly 100 is manufactured, in pertinent part, by the following steps. First, electrodes 104 and 106 and traces 112, 114 and 130 are printed onto a polymeric backing, such as treated ink-printable PET film, for example, or another suitably rigid material. The dielectric layer 118 may then be deposited onto the appropriate portions of traces 112 and 114 that are not intended to electrically contact the electrode reservoirs and contacts of an interconnect between the electrode assembly and a power supply/controller, for example. The polymeric backing onto which the electrodes are printed is then laminated to the flexible backing 108. The anode reservoir 134 and cathode reservoir 135 are then positioned onto the electrodes 104 and 106, respectively. In the assembly of the release cover 138, the transfer absorbents 144 and 146 are ultrasonically spot welded within wells 140 and 142 and are loaded with an appropriate loading solution for absorption and/or diffusion into the anode and/or cathode reservoirs 134 and 136. An excess of about 5% loading solution (over the amount needed to absorb and diffuse into the hydrogel) typically is added to the reservoir covers due to in the about 95% transfer efficiency of the loading process, resulting in some of the loading solution remaining in the absorbent reservoir covers.

Once assembled and loaded with loading solution, the release cover is positioned on the electrode assembly 100 with the loaded transfer absorbents 144 and 146 in contact with anode and cathode reservoirs 134 and 136, respectively. Over a time period, typically at least about 24 hours, substantial portions (about 95%) of the loading solutions are absorbed and diffused into the hydrogel reservoirs. The completed assembly is then packaged in an inert gas environment and hermetically sealed.

In one method of use, the release cover 138 is removed from the electrode assembly 100, and the electrode assembly 100 is placed on a patient's skin at a suitable location. After the electrode assembly 100 is placed on the skin, it is inserted into a suitable interconnect, such as a component of the connector assembly 250, for example. An electric potential is applied according to any profile and by any means for electrically assisted drug delivery known in the art. Examples of power supplies and controllers for electrically assisted drug delivery are well known in the art, such as those described in U.S. Patent Nos. 6,018,680 and 5,857,994, among others. Ultimately, the optimal current density, drug concentration and duration of the electric current and/or electric potential is determined and/or verified experimentally for any given electrode/electrode reservoir combination.

The electrodes described herein are standard Ag or Ag/AgCl electrodes and can be prepared in any manner according to standard methods in such a ratio of Ag to AgCl (if initially present), thickness and pattern, such that each electrode will support the electrochemistry for the desired duration of treatment. Typically, as is common in preparation of disposable iontophoresis electrodes, the electrodes and electrode traces are prepared by printing Ag/AgCl ink in a desired pattern on a stiff polymeric backing, for example 2 mm PET film, by standard lithographic methods, such as by rotogravure. Ag/AgCl ink is commercially availab'e from E.I. du Pont de Nemours and Company, for example and without limitation, du Pont Product ID Number 5279. The dielectric also may be applied to the electrode traces by standard methods. As with the electrode, dielectric ink may be applied in a desired pattern over the electrodes and electrode traces by standard printing methods, for instance by rotogravure.

The pressure-sensitive adhesive (PSA) and transfer adhesives may be any pharmaceutically acceptable adhesive suitable for the desired purpose. In the case of the pressure-sensitive adhesive, the adhesive may be any acceptable adhesive useful for affixing an electrode assembly to a patient's skin or other membrane. For example, the adhesive may be polyisobutylene (PIB) adhesive. The transfer adhesive, used to attach different layers of the electrode assembly to one another, also may be any pharmaceutically acceptable adhesive suitable for that purpose, such as PIB adhesive. For assembly of the electrodes described herein, the PSA typically is provided pre-coated on the backing material with a silicone-coated release liner attached thereto to facilitate cutting and handling of the material. Transfer adhesive typically is provided between two layers of silicone-coated release liner to facilitate precise cutting, handling and alignment on the electrode assembly.

The anode and cathode reservoirs described herein may comprise a hydrogel. The hydrogel typically is hydrophilic and may have varying degrees of cross-linking and water content, as is practicable. A hydrogel as described herein may be any pharmaceutically and cosmetically acceptable absorbent material into which a loading solution and ingredients therein can be absorbed, diffused or otherwise incorporated and that is suitable for electrically assiste 1 drug delivery. Suitable polymeric compositions useful in forming the hydrogel are known in the art and include, without limitation, polyvinylpyrrolidone (PVP), polyethyleneoxide, polyacrylamide, polyacrylonitrile and polyvinyl alcohols. The reservoirs may contain additional materials such as, without limitation: preservatives, such as Phenonip Antimicrobial, available commercially from Clariant Corporation of Mount Holly North Carolina; antioxidants, such as sodium metabisulfite; chelating agents, such as EDTA; and humectants. A typical unloaded reservoir contains preservatives and salt. As used herein in reference to the water component of the electrode reservoirs, the water is purified and preferably meets the standard for purified water in the USP XIV.

As discussed above, the hydrogel has sufficient internal strength and cohesive structure to substantially hold its shape during its intended use and leave essentially no residue when the electrode is removed after use. As such, the cohesive strength of the hydrogel and the adhesive strength between the hydrogel and the electrode are each greater than the adhesive strength of the bonding between the hydrogel and the membrane (for instance skin) to which the electrode assembly is affixed in use.

The donor (anode) reservoir also includes a salt, preferably a fully ionized salt, for instance a halide salt such as sodium chloride in a concentration of from about 0.001 wt % to about 1.0 wt. %, preferably from about 0.06 wt. % to about 0.9 wt. %. The salt content is sufficient to prevent electrode corrosion during manufacture and shelf-storage of the electrode assembly. These amounts may vary for other salts in a substantially proportional manner depending on a number of factors, including the molecular weight and valence of the ionic constituents of each given salt in relation to the molecular weight and valence of sodium chloride. Other salts, such as organic salts, are useful in ameliorating the corrosive effects of certain drug salts. Typically the best salt for any ionic drug will contain an ion that is the same as the counter ion of the drug. For instance, acetates would be preferred when the drug is an acetate form. However, the aim is to prevent corrosion of the electrodes.

Lidocaine HCl and epinephrine bitartrate are used in the examples below to elicit a desired pharmacological response. If the counterion of lidocaine is not chloride, though chloride ions may be useful to prevent electrode corrosion, a corrosion-inhibiting amount of that other counterion may be present in the unloaded reservoir in addition to, or in lieu of the chloride ions to prevent corrosion of the electrode. If more than one counterion is present, such as in the case where more than one drug is loaded and each drug has a different counterion, it may be preferable to include sufficient amounts of both counterions in the reservoir to prevent electrode corrosion. It should be noted that in the examples provided below, the amount of epinephrine bitartrate loaded into the gel is not sufficient to cause corrosion.

The return (cathode) reservoir may be a hydrogel with the same or different polymeric structure and typically contains a salt such as sodium chloride, a preservative and, optionally, a humectant. Depending upon the ultimate manufacturing process, certain ingredients may be added during cross-linking of the hydrogel reservoir, while others may be loaded with the active ingredients. Nevertheless, it should be recognized that irrespective of the sequence of addition of ingredients, the salt must be present in the reservoir adhering to the electrode and substantially evenly distributed therethrough prior to the loading of the active ingredient(s) or other ingredient that causes formation of concentration cells.

As used herein, "stable" and "stability" refer to a property of individual packaged electrode-reservoir assemblies, and typically is demonstrated statistically. The term "stable" refers to retention of a desired quality, with particular, but not exclusive focus on active ingredients such as epinephrine content, lidocaine content, hydrogel strength, hydrogel tack, electrical circuitry and electrical capacity, within a desired range. For example, in an iontophoretic device, the U.S. Food and Drug Administration (FDA) may require retention, as a lot, of 90% of the label claim of epinephrine over a given time period using a least square linear regression statistical method with a 95% confidence level. However, as used herein, an electrode assembly and/or parts thereof, are considered stable so long as they substantially retain their desired function in an iontophoretic system. Stability, though measured by any applicable statistical method, is a quality of the electrode assembly. Therefore, methods other than FDA-approved statistical methods may be used to quantitate stability. For instance, even though for FDA purposes, a 95% confidence level may be required, those limits are not literally required for a device to be called "stable." Similarly, and for exemplary purposes only, a "stable" iontophoretic electrode may be said to retain 80% of the original epinephrine concentration over a given time period, as determined by least square linear regression analysis.

As used generally herein, an electrode-reservoir, reservoir or electrode assembly is stable when hermitically sealed for a given time period. This means that when the electrode assembly is sealed in a container that is impermeable to oxygen and water ("hermetically sealed"), the electrode-reservoir retains a specified characteristic or parameter within desired boundaries for a given time period. By "original concentration", "original amounts" or "original levels" it is meant the concentration, amount or level of any constituent or physical, electrochemical or electrical parameter relating to the electrode assembly at a time point designated as t=0, and typically refers to a time point after the electrode assembly is sealed within the hermetically sealed container. This time may take up to a few weeks to ensure uniform distribution of ingredients in the reservoir(s).

As briefly mentioned above, "stability" may refer to a variety of qualities of the reservoir-electrode. Drug or pharmaceutical stability is one parameter. For instance, epinephrine typically is very unstable. Therefore, an iontophoretic electrode assembly might be considered stable for the time period that useful quantities of epinephrine remain available for delivery. Similarly, if lidocaine is considered, the electrode assembly remains stable for the time period that useful quantities of lidocaine remain available for delivery.

Physical stability also may be considered. Hydrogel strength (for example, apparent compressive modulus, as shown in the Examples) and probe tack are examples of the parameters considered for physical stability. In the case of electrical and/or electrochemical stability, retention of useful current capacity (specific capacity; $mA\text{-}min/cm^2$) may be measured. As discussed above, though the FDA requires specific statistical tests and limits to permit an iontophoretic device to be marketed as stable, those standards are examples of what is considered to be a stable parameter, stability referring to retention of a parameter within desired boundaries to remain functional. This typically is a range of given properties, for example as shown in the Examples below.

Described with specificity herein is an embodiment of an iontophoretic system for delivery of the topical anesthetic lidocaine with the vasoconstrictor epinephrine, more specifically lidocaine HCl and epinephrine bitartrate as shown in the Examples. The particular amounts of epinephrine and lidocaine shown in the Examples are selected to produce effective local anesthesia. Variations in the relative concentration and/or mass of lidocaine and/or epinephrine, as well as variations in reservoir volume, reservoir composition, reservoir skin contact surface area, electrode size and composition and electrical current profile, among other parameters, could result in changes in the optimal concentrations of lidocaine and/or epinephrine in the gel reservoir. A person of skill in the art would be able to adjust the relative amounts of ingredients to achieve the same results in a system in which any physical, electrical or chemical parameter differs from those disclosed herein.

For most, if not all applications, epinephrine stability should not be dependent upon epinephrine concentration within a range that can be extrapolated from the data provided herein. A useful range of epinephrine is, therefore, from about 0.01 mg/ml to about 3.0 mg/ml.

Although lidocaine is a common topical anesthetic, other useful topical (surface and/or infiltration) anesthetics may be used in the described system. These anesthetics include, without limitation, salts of: amide type anesthetics, such as bupivacaine, butanilicaine, carticaine, cinchocaine/dibucaine, clibucaine, ethyl parapiperidino acetylaminobenzoate, etidocaine, lidocaine, mepivicaine, oxethazaine, prilocaine, ropivicaine, tolycaine, trimecaine and vadocaine; ester type anesthetics, including esters of benzoic acid such as amylocaine, cocaine and propanocaine, esters of metaaminobenzoic acid such as clormecaine and proxymetacaine, esters of paraaminobenzoic acid (PABA) such as, amethocaine (tetracaine), benzocaine, butacaine, butoxycaine, butyl aminobenzoate, chloroprocaine, oxybuprocaine, parethoxycaine, procaine, propoxycaine and tricaine; and miscellaneous anesthetics, such as, bucricaine, dimethisoquin, diperodon, dyclocaine, ethyl chloride, ketocaine, myrtecaine, octacaine, pramoxine and propipocaine.

Of the topical anesthetics, salts of bupivacaine, butacaine, chloroprocaine, cinchocaine, etidocaine, mepivacaine, prilocaine, procaine, ropivacaine and tetracaine (amethocaine) might be considered by some to be more clinically relevant than other anesthetics listed above, though not necessarily more effective. Certain other features of each of the compounds listed above may make any particular compound more or less suited to iontophoretic delivery as described herein. For example, use of cocaine may be contra-indicated because of its cardiovascular side effects. Bupivacaine, butacaine, chloroprocaine, cinchocaine, etidocaine, mepivacaine, prilocaine, procaine, ropivacaine and tetracaine (amethocaine) may be preferred as substitute for lidocaine because the all have similar pKs of about 8 or >8, meaning they will ionize under the same conditions as lidocaine. Iontophoresis *in vitro* across human skin has shown that bupivacaine and mepivacaine show a similar cumulative delivery as lidocaine, while etidocaine, prilocaine and procaine have shown slightly greater delivery. Chloroprocaine, procaine and prilocaine have similar relatively short duration effects (< 2 hr) whereas bupivicaine, etidocaine, and mepivacaine have effects lasting 3-4 hr. These times are approximately doubled when epinephrine is used in conjunction with these anesthetics. The duration of the action of the local anesthetic is dependent upon the time for which it is in contact with the nerve. This duration of effect will depend on the physiochemical and pharmacokinetic properties of the drug. Hence, any procedure that can prolong contact between the therapeutic agent and the nerve, such as co-delivery of a vasoconstrictor with the anesthetic, will extend the duration of action.

Another factor that should be considered is that ester-based anesthetics based on PABA are associated with a greater risk of provoking an allergic reaction because these esters are metabolized by plasma cholinesterase to yield PABA, a known allergen. For this reason, amide anesthetics might be preferred and molecules such as chloroprocaine, and procaine would not be viewed as first-line replacements for lidocaine. Because bupivacaine, etidocaine, mepivacaine, ropivicaine and prilocaine are amide anesthetics with similar physiochemical properties and clinical effects as lidocaine, they may be preferred by some as substitutes for lidocaine. A secondary issue with prilocaine is that although it is generally considered to be the safest of the amide anesthetics, one of its metabolites (o-toluidine) has been associated with increased risk of methemoglobinemia and cyanosis as compared to the other amide anesthetics.

Each of the anesthetics listed above have varying degrees of vasoconstrictor activity. Therefore, optimal concentrations of the anesthetic and the vasoconstrictor will vary depending on the selected local analgesic. However, for each local anesthetic, optimal effective concentration ranges can be readily determined empirically by functional testing. As used herein, the terms "anesthetic" and -"anesthesia" refer to a loss of sensation, and are synonymous with "analgesics" and "analgesia" in that a patient's state of consciousness is not considered when referring to local effects of use of the described iontophoretic device, even though some of the drugs mentioned herein may be better classified as "analgesics" or "anesthetics" in their systemic use. Sodium metabisulfite may be added to the donor reservoir to scavenge oxygen. The amount of sodium metabisulfite added is not substantially in excess of the amount needed to scavenge all oxygen from the packaged reservoir for a given time period to minimize the formation of the adduct epinephrine sulfonic acid, and other decomposition products. For example, the donor hydrogel may contain less than about 110%, for example about 101%, of the amount of sodium metabisulfite equal to a minimal amount of sodium metabisulfite needed to scavenge substantially all oxygen in the packaged donor hydrogel. The amount of sodium metabisulfite needed to scavenge oxygen in the packaged donor hydrogel for any given amount of time can be calculated from the amount of oxygen present within the package in which the donor hydrogel is hermetically sealed. Alternately, the optimal amount of sodium metabisulfite can be titrated by determining the amount of sodium metabisulfite at which production of the oxidation products of epinephrine, due to its reaction with oxygen, such as adrenolone or adrenochrome, and epinephrine sulfonic acid essentially stops.

**Examples**

**Example 1 - Preparation of electrode assembly**

The following components were assembled to prepare an electrode assembly, essentially as shown in the figures discussed above, for delivery of lidocaine and epinephrine by iontophoresis.

**Backing**: ethylene vinyl acetate (EVA) (4.0 mil ± 0.4 mil) coated with polyisobutylene (PIB) adhesive (6 mg/cm$^2$), (Adhesive Research of Glen Rock, Pennsylvania). The backing was dimensioned to yield a gap of between 0.370 inches and 0.375 inches ± 0.005 inches between the gel electrode and the outer edge of the backing at any given point on the edge of the gel. Excluding the tactile feedback notch and the wings, the tab end of the electrode had a width of 0.450 inches to 0.500 inches ± 0.005 inches.

**Tab stiffener**: 7 mil PET/acrylic adhesive (Scapa Tapes of Windsor Connecticut).

**Printed electrode**: Ag/AgCl electrode printed on du Pont 200 J102 2 mil clear printable PET film with dielectric coated Ag/AgCl traces. The Ag/AgCl ink was prepared fro n du Pont Ag/AgCl Ink #5279, du Pont Thinner #8243, du Pont Defoamer and methyl amyl ketone (MAK). The dielectric ink was Sun Chemical Dielectric Ink # ESG56520G/S. The electrodes were printed by rotogravure substantially as shown in Figures 1 and 2, with a coatweight of both the electrode ink and the dielectric ink of at least about 2.6 mg/cm$^2$. The anode had a diameter of 0.888 inches ± 0.005 inches. The cathode was essentially oval shaped, as shown in the figures. The semicircular ends of the oval both had a radius of 0.193 inches ± 0.005 inches. The cente-s of the semicircular ends of the oval were separated by 0.725 inches ± 0.005 inches.

**Transfer Adhesive**: 6 mg/cm$^2$ ± 0.4 mg/cm$^2$ Ma-24A PIB transfer adhesive, (Adhesives Research). When printed onto the electrode, there was a gap of 0.030 inches ±0.0030 inches between the anode and cathode electrodes and the transfer adhesive surrounding the electrodes.

**Anode Gel Reservoir**: 40 mil high adhesion crosslinked polyvinylpyrrolidone (PVP) hydrogel sheet containing. 24% wt. ± 1% wt. PVP; 1% wt. ± 0.05% wt. Phenonip; 0.06% wt. NaCl to volume (QS) with purified water (USP).

The hydrogel was crosslinked by electron beam irradiation at an irradiation dose of about 2.7 Mrad (27 kGy) at an electron beam voltage of 1 MeV. The anode gel reservoir was circular, having a diameter of 0.994 inches ± 0.005 inches and has a volume of about 0.8 mL (0.7 g). The reservoir was loaded by placing 334 mg of Loading Solution A, onto the absorbent (non-woven), described below, and then placing the cover assembly containing the absorbent onto the patch so that the absorbent contacts the anode reservoir directly, permitting the loading solution to absorb into the reservoir.

Loading Solution A was prepared from the ingredients shown in Table A, resulting in an anode reservoir composition as presented in Table B.

Table A -

| Loading Solution A | |
|---|---|
| **Ingredient** | **% Wt.** |
| Lidocaine hydrochloride USP | 30 |
| L-epinephrine bitartrate USP | 0.5725 |
| NaCl | 0.06 |
| Disodium EDTA | 0.03 |

Table A - (continued)

| Loading Solution A | |
|---|---|
| **Ingredient** | **% Wt.** |
| Citric acid | 0.06 |
| Glycerin | 30 |
| Sodium metabisulfite | 0.15 |
| Purified Water | QS |

Table B -

| Anode Reservoir Composition | | |
|---|---|---|
| **INGREDIENT** | **mg/Reservoir** | **FUNCTION** |
| Lidocaine HCL monohydrate, USP | 100 | Anesthetic |
| L-epinepbrine bitartrate, USP | 1.90,1.05 as free base | Vasoconstrictor |
| Glycerin | 100 | Humectant |
| Sodium Chloride | 0.52 | Anti-corrosion Agent |
| Sodium Metabisulfite | 0.5 | Antioxidant |
| Edetate Disodium | 0.1 | Chelating Agent |
| Citric Acid | 0.2 | Antioxidant Synergist, Chelating Agent |
| Phenoxy ethanol + Parabens | 5.3 | Preservative |
| Water | 530 | Vehicle, Mobile Phase |
| PVP | 138 | Physical Structure |
| * 1.05 mg as free base. | | |

**Cathode Reservoir**: The unloaded cathode gel consisted of a 40 mil high adhesion polyvinylpyrrolidone (PVP) hydrogel sheet containing: 24% ± 1% wt. PVP, 1% Phenonip antimicrobial, 0.06 % wt. NaCl and purified water (Hydrogel Design Systems, Inc.) The hydrogel was crosslinked by electron beam irradiation at an irradiation dose of about 2.7 Mrad (27 kGy) at an electron beam voltage of 1 MeV. The cathode reservoir was essentially oval shaped, as shown in the figures. The semicircular ends of the oval both had a radius of 0.243 inches ± 0.005 inches. The centers of the semicircular ends of the oval were separated by 0.725 inches ± 0.005 inches and the volume of the cathode reservoir was about 036 mL (0.37 g). The cathode reservoir was loaded by placing 227 mg of cathode loading solution, described below onto the absorbent (non-woven) described below and then placing the cover assembly containing the absorbent onto the patch so that the absorbent contacts the cathode reservoir directly, permitting the loading solution to absorb into the reservoir. Cathode loading Solution was prepared from the ingredients shown in Table C, resulting in a cathode reservoir composition as presented in Table D.

Table C-

| Cathode Loading Solution | |
|---|---|
| **Ingredient** | **% Wt.** |
| Glycerin | 30 |
| NaCl | 1.28 |
| Phenoxyethanol-parabens mixture | 0.10 |
| Sodium Phosphate monobasic | 6.23% |
| Water | QS |

Table D -

| Cathode Reservoir Composition | | |
|---|---|---|
| INGREDIENT | mg/ Patch | FUNCTION |
| Glycerin | 68.3 | Humectant |
| Sodium Chloride | 3 | Anti-corrosion Agent |
| Monobasic Sodium Phosphate | 14.2 | Acidulating Agent |
| Phenoxy ethanol + Parabens | 3.3 | Preservative |
| PVP | 89 | Physical Structure |
| Water | 419 | Vehicle, Mobile Phase |

Within-lot variation in solution doses and composition typically is ±5%, but has not been analyzed statistically.

**Release cover**: 7.5 mil ± 0.375 mil polyethylene terephthalate glycolate (PETG) film with silicone coating (Furon 7600 UV-curable silicon).

**Nonwoven**: 1.00 mm ± 0.2 mm Vilmed M1561 Medical Nonwoven, a blend of viscose rayon and polyester/ polyethylene (PES/PE) fibers thermal-bonded to PE (Freudenberg Faservliesstoffe KG Medical Nonwoven Group of Weinheim, Germany).

**Electrode Assembly**: The electrode was assembled substantially as shown in the figures, with the anode and cathode reservoirs laminated to the electrodes. The tab stiffener was attached to the tab end of the backing of electrode assembly on the opposite side of the backing from the anode and cathode traces. The drugs were added to the unloaded anode reservoir as indicated below.

**Packaging**: The assembled electrode assembly was hermetically sealed in a foiJ.lined polyethylene pouch purged with nitrogen gas.

**Example 2 - Preparation of hydrogel electrode reservoirs - droplet loading**

In another embodiment, unloaded gel reservoirs within an integrated patch assembly were prepared as follows to the specifications shown in Table E:

Table E

| Ingredient | % Wt. |
|---|---|
| PVP | 24.0 |
| Phenonip antimicrobial (phenoxy ethanol and parabens) | 1.0 |
| NaCl | 0.06 |
| Purified water | QS |

The gels were crosslinked by electron beam irradiation at an irradiation dose of about 2.7 Mrad (27 kGy) at an electron beam voltage of 1 MeV.

The unloaded anode gel reservoirs were placed on Ag/AgCl anodes and 0.32 ml aliquots of Loading Solution A (Table A) were placed on the reservoirs and were permitted to absorb and diffuse into the reservoir.

**Example 3 - Stability Study**

The following examples provide a complete description of the three stability lots (lots 1, 2 and 3) of 5,000 patches prepared according to Example 1, with stability data from samples at four storage conditions, as indicated in TABLE F:

TABLE F -

| Reported Stability Time/Storage Conditions | |
| --- | --- |
| **Time** | **Storage Conditions** |
| 24 months | 5°C |
| 24 months | 25°C/60% RH (relative humidity) |
| 12 months | 30°C/60% RH |
| 6 months | 40°C/75% RH |

The following represents 24 month data at 5°C, 24 month data at 25°C/60% RH, 12 month data at 30°C/60% RH and six months stability data at 40°C/75% RH on lots 1, 2, and 3. Stability test methods and specifications are described below. PVP- gel reservoirs were prepared according to Example 1.

**Test Methods and Specifications**

The stability specifications and analytical test methods are provided as follows:

**Test Method A**

**HPLC Method Lidocaine Hydrochloride**

Lidocaine hydrochloride, which is contained in the anode drug (dispensing) solution and in the anode hydrogel, is measured directly from the solution or is extracted from the anode hydrogel reservoir. Lidocaine is removed from the anode hydrogel reservoir during extraction in a 0.01 M acetate buffer solvent, (pH 3.8) followed by HPLC analysis using a Waters C8 column with a UV detector at 254 nm. Lidocaine is reported as lidocaine hydrochloride. The analysis uses a linear gradient mobile phase of acetonitrile/acetate buffer ranging from 80/20 to 60/40 throughout the run. The concentration of the working standard is approximately 0.041 mg/mL. Essentially the same chromatography is employed in the analysis of lidocaine in the anode loading solution, where the method is run for seven minutes isocraticahy using 80/20 acetonitrile/0.01 M acetate buffer mobile phase (pH 3.8).

**HPLC Method Epinephrine Bitartrate**

Epinephrine bitartrate is added to the loading (dispensing) solution and is contained within the anode hydrogel reservoir. As with lidocaine, it is measured directly in the loading solution or extracted from the anode hydrogel reservoir prior to analysis. Epinephrine bitartrate in the anode hydrogel is extracted simultaneously with lidocaine using the same extraction with 0.01 M acetate buffer solvent, (pH 3.8). However, the chromatography is different Epinephrine is measured by HPLC analysis of the extract using a Waters Nova-Pak® C18 column with an UV detector set at 280 mn and is reported as epinephrine free base. The analysis uses a linear gradient mobile phase of 0.05 M phosphate buffer/ methanol mobile phase (pH 3.8) with concentrations from 85/15 to 15/85 throughout the run. The concentration of the working standard in this analysis is 0.02 mg/mL.

**Test Method B**

**HPLC Assay Method for Lidocaine Degradation Products In Iontophoretic Patches and Anode Loading Solution**

The most likely degradation product for lidocaine is 2,6-Dimethylaniline. It has never been detected in the drug product during the normal stability storage conditions or during forced degradation studies. This and other potential degradants can be analyzed by HPLC using a Waters Nova-Pak® C8 column with an UV detector set at 254 nm. For the analysis, the entire patch configuration is extracted for three hours in an acetate buffer/acetonitrile solvent (pH 3.4).

**Test Method C**

**HPLC Method Epinephrine Degradants - Epinephrine Sulfonic Acid and Adrenalone**

These compounds have been identified as the two main products expected to form with degradation of epinephrine. Epinephrine sulfonic acid is the addition product of epinephrine and sodium metabisulfite and adrenalone is the oxidation product of epinephrine. Other potential degradation products were initially considered, however, during forced degradation studies, the above two products were the only degradation products identified. For example, Adrenochrome was initially considered as a potential degradation product, however, studies showed that this degradant was unstable and quickly polymerized. The method employs an HPLC method for the quantitation of these potential degradants at the 0.1% (of Epinephrine in the finished patch) level. The degradants are extracted from the anode hydrogel reservoir for three hours in an acetate buffer with 5% acetonitrile. The method uses an electrochemical detector: DC amperometry mode, +0.70 V potential, 2 µA range and a Waters SymmetryShield™ RP8 chromatographic column (equivalent to USP packing L7). The gradient analysis is run for 55 minutes starting with 100% mobile phase B and transitioning through 10/90 acetate buffer (pH = 3.8)/acetonitrile back to 100% mobile phase B (acetate buffer with 5% acetonitrile).

**Test Method D**

**HPLC Method Preservative - Phenonip®**

The Phenonip components (2-phenoxyethanol, methyl-, ethyl-, propyl-, butyl-and isobutyl-parabens) in the anode and cathode hydrogel reservoirs and in the cathode loading (dispensing) solution are analyzed by HPLC. This isocratic analysis is performed using a UV detector set at 270 nm with a Waters Symmetry® C18 column, and a (0.05M) phosphate buffer/acetonitrile mobile phase (35/65) at a pH of 3.8. The Phenonip components are extracted from the hydrogels prior to analysis. Working standards are used as reference for all ingredients in the preservative.

**Test Method E**

**pH of Hydrogel Surface**

pH of hydrogel surfaces were measured using an ATI Orion PerpHect® Meier, Model 370 and an Orion Flat Surface PerpHect® Combination pH Electrode 0-14 pH, epoxy body, model 8235BN.

**Test Method F**

**Surface Texture and Compressive Modulus Analysis of Hydrogel Reservoirs and Peripheral Adhesive in Lidocaine Iontophoretic Patch System**

The purpose of this test is measure the strength of the anode and cathode hydrogel reservoirs as well as the tack properties of these components in the lidocaine iontophoretic patch. The test is also utilized in the determination of the tack properties of the peripheral adhesive in the finished patch. A texture analyzer (Model TA-XT 2i, Texture Technologies, Scarsdale, N.Y.) was chosen to measure both tack and strength of the skin contacting components of the patch. The texture analyzer measures both force and displacement penetrating the surface of a material and upon removal. A small diameter probe is used with this instrument. Multiple readings on all skin contacting surfaces in the patch can be measured without disassembling the patch. The apparent compressive modulus can also be measured using this instrument since the texture analyzer can be programmed to operate at a given constant penetration force, deformation rate, dwell and removal rate. For testing of the gel, penetration force was 50 g, deformation rate was 0.1 cm/s, dwell was 30 s and the removal rate was 1 cm/s. The adhesive testing was conducted in the same manner, except the dwell was about 1 s.

**Test Method G**

**Aerobic Plate Count**

The aerobic plate count was conducted according the standards of USP<61>.

**Test Method H**

**Procedure to Evaluate Anode and Cathode Specific Capacity for Printed Electrode Material**

Specific capacity is a measure of the amount of material available electrochemically to sustain iontophoretic drug delivery. To perform the test, an electrochemical cell is formed by attaching an iontophoretic patch, containing Ag/AgCl electrodes, to an ionically conducting agarose gel. A specified constant direct current is applied to the test cell using a power supply. The constant current output from the power supply is set using a calibrated ammeter. Anode and cathode potentials and the current are monitored continuously using calibrated instruments. The test is run until the anode and cathode potentials reach voltage endpoints related to the Ag/AgCl electrode reaction. The specific capacity is calculated from the applied current, time to reach the voltage endpoints and the electrode area.

**Test Method I**

**Measurement of the Dielectric Leakage Current**

The dielectric leakage current is a measure of the parasite current through the dielectric coating that may arise if ihe conductive traces contact a conducting medium. To measure the dielectric leakage current, a circuit is constructed by connecting two dielectric coated conductive ink traces with an ionically conducting hydrogel (0.06 % wt. sodium chloride). A constant current is applied to the circuit and the resultant current, the dielectric leakage current, is directly measured with an ammeter. The leakage current per unit length is determined by dividing the dielectric leakage current by the length of the dielectric which is covered by the hydrogel. All of the measurements were made using calibrated electronic equipment.

**Test Method J**

**Measurement of Patch Leakage Current**

The purpose of this test is to detect a parasitic current in the patch that might arise from an ionic pathway between the anode and cathode electrodes. The method is based upon a straightforward application of Ohm's Law. A simple circuit is constructed that consists of a constant voltage to the anode and cathode leads and the resultant current, the patch leakage current, is directly measured with an ammeter.

**Test Method K**

**Trace Conductance**

The purpose of this method is to characterize the integrity of the electrical path through the conductive traces in the patch. The integrity of the conductive traces affects power consumption of the controller power source and in the worst case scenario. A break in the conductive trace would lead to a non-operable system. The trace conductance in measured using a standard AC impedance electronic instrument (LCR Meter) by measuring directly the resistance (conductance) between the electrode tab and the trace interconnect tab.

**Test Method L**

**Procedure to Evaluate Hydrogel-Electrode Conductivity**

The purpose of the method is to characterize the integrity of the electrical path through the electrode-gel assemblies in the patch. The integrity of the electrode-gel assembly affects the quantity and uniformity of drug delivered. This property is characterized by measuring the conductivity.
To perform the test, an electrochemical cell is formed by attaching a counter electrode to the electrode-gel assembly of the iontophoretic patch. The resistance of the electrochemical cell is measured using a standard AC impedance electronic instrument (LCR meter). The resistances of the interconnect traces and electrode-gel assemblies are measured. Also, the thickness of the electrochemical cell is measured and the electrode-gel conductivity's then calculated from the above measurements.

**Test Method M**

**Measurement of Pouch Opening Force**

Pouch opening force for sealed pouches was measured using an Insertion tensile tester, Model 5565 with a 50N capacity static load cell and pneumatic air grips. This type of est is well known in the packaging art for use in testing foil laminate packaging material.

**Test Method N**

**Measurement of Pouch Burst Strength**

Burst strength of the pouches was measured with a TM Electronics BT-1000-15 package tester. This type of test also is well known in the packaging art for use in testing foil laminate packaging material.

Table G provides a summary of specification ranges for the tested lots, as measured at time (t) = 0.

Table G -

| Summary of Stability Test Methodology and Specifications | | |
|---|---|---|
| **Test** | **Test Method** | **Specification** |
| **ANODE RESERVOIR** | | |
| **Drug Content** | | |
| Lidocaine HCl | A | 85.5 - 104.5 mg/patch |
| Epinephrine (Free Base Assay) | | 0.85 - 1.10 mg/patch |
| ***Degradants*** | | |
| Lidocaine Degradants | | |
| Individual Unidentified | B | ≤200 ug/patch |
| Total Degradants | | ≤200 ug/patch |
| Epinephrine Degradants | | |
| Adrenalone | C | ≤10 ug/patch |
| Epinephrine Sulfonic Acid | | ≤100ug/patch |
| Individual Unidentified | | ≤5 ug/patch |
| Total Degradants | | ≤150 ug/patch |
| Total Degradants (Lidocaine + Epinephrine) | B, C | ≤350 ug/patch |
| Preservative Assay | D | ≥3.0 mg/g |
| pH Hydrogel Surface | E | 3.7-4.5 |
| ***Physical*** | | |
| Probe Tack | F | Avg. ≥6 g |
| | | Min. ≥4 g |
| Apparent Compressive Modulus | | ≥0.6 g |
| ***Microbial limits*** | | |
| Total Aerobic Plate Count | G | ≤100 CFU/reservoir[1] |
| **CATHODE RESERVOIR** | | |
| Preservative Assay | D | ≥3.0 mg/g |
| pH Hydrogel Surface | E | 4.0 - 6.0 |

[1] None detected for Anaerobes, *Pseudomonas aeruginosa, Staphylococcus aureus, Escherichia coli, Salmonella sp., Clostridium perfringens.*

Table G -   (continued)

| Summary of Stability Test Methodology and Specifications | | |
|---|---|---|
| **Test** | **Test Method** | **Specification** |
| *Physical* | | |
| Probe Tack | F | Avg. ≥4 g |
| | | Min. ≥3 g |
| Apparent Compressive Modulus | | ≥0.6 g |
| *Microbial Limits* | | |
| Total Aerobic Plate Count[1] | G | ≤100 CFU/reservoir[1] |

| PATCH | | |
|---|---|---|
| *Physical* | | |
| Probe Tack (Peripheral adhesive) | F | Avg. ≥150 g |
| | | Min. ≥50 g |
| *Electrochemical/Electrical Properties* | | |
| **Specific Capacity** | | |
| ·    Anode | H | Avg. ≥6.7 mA-min/cm$^2$ |
| | | Min. ≥5.6 mA-min/cm$^2$ |
| ·    Cathode | H | ≥7.4mA-min/cm$^2$ |
| Dielectric Leakage Current | I | Avg. ≤44.4 uA/in |
| | | Max. ≤55.5 uA/in |
| Patch Leakage Current | J | ≤0.62 UA @ 35 V$^-$ |
| **Patch Conductance** | | |
| Trace Conductance | | |
| ·    Anode | K | (ohm) $^{-1}$ ≥0.001 |
| ·    Cathode | K | ≥0.001 (ohm)$^{-1}$ |
| **Hydrogel/Electrode conductivity** | | |
| ·    Anode | L | Avg. ≥0.0050 (ohm-cm)$^{-1}$ |
| | | Min. ≥0.0042 (ohm-cm)$^{-1}$ |
| ·    Cathode | L | Avg. ≥0.0031 (ohm-cm)$^{-1}$ |
| | | Min. ≥0.0028 (ohm-cm)$^{-1}$ |

| CONTAINER CLOSURE | | |
|---|---|---|
| Opening Force | M | 1000 - 2400 g |
| Burst Test | N | 6 - 18 psi |

## A. Stability Data 5°C

Refrigerated storage stability data on lots 1, 2 and 3 stored at 5°C are contained within Tables H, I and J, respectively. All data are within the proposed specifications at all time points through 24 months. There appear to be no adverse effects on the patch or foil/foil pouch attributable to the low temperature storage. The data may be used to support temperature excursions beyond those permitted by the labeling.

**Table H: Refrigerated Temperature (5°C) Stability Data    Lot Number 1**

**95.0 mg Lidocaine HCl/ 1.0 mg Epinephrine free base Iontophoretic patch**

| Test – Anode Reservoir | Method | Specification | Time in Months | | | | | | |
|---|---|---|---|---|---|---|---|---|---|
| | | | 0 | 3 | 6 | 9 | 12 | 18 | 24 |
| **Drug Assay** | | | | | | | | | |
| Lidocaine HCl Assay | A | 85.5 – 104.5 mg/patch | 102.3 | 102.3 | N/A | 100.9 | 97.4 | 99.0 | 96.5 |
| Epinephrine Assay | | 0.85 – 1.10 mg/patch | 1.06 | 1.02 | 1.04 | 1.03 | 1.03 | 1.02 | 1.03 |
| Lidocaine Degradants | | | | | | | | | |
| Individual Unidentified Degs | B | ≤200 ug/patch | ND | ND | ND | N/A | ND | ND | ND |
| Total Lidocaine Degs | | ≤200 ug/patch | ND | ND | ND | N/A | ND | ND | ND |
| Epinephrine Degradants | | | | | | | | | |
| Epinephrine Sulfonic Acid | C | ≤100 ug/patch | 4.4 | 5.8 | 6.0 | 6.8 | 7.6 | 6.7 | 6.6 |
| Adrenalone | | ≤10 ug/patch | ND | ND | ND | ND | ND | ND | ND |
| Individual Unidentified Degs | | ≤5 ug/patch | ND | ND | ND | ND | ND | ND | ND |
| Total Epinephrine Degs | | ≤150 ug/patch | 4.4 | 5.8 | 6.0 | 6.8 | 7.6 | 6.7 | 6.6 |
| **Total Degradants** | B C | ≤350 ug/patch | 4.4 | 5.8 | 6.0 | 6.8 | 7.6 | 6.7 | 6.6 |
| Preservative Assay | D | ≥3.0 mg/g | | NA | NA | NA | 5.0 | N/A | 5.1 |
| pH Hydrogel Surface | E | 3.7 - 4.5 | 4.0 | 4.1 | 4.1 | 4.2 | 4.2 | 4.1 | 4.2 |
| Probe Tack | F | Avg. ≥6 g | 13 | 10 | 11 | 16 | 12 | 10 | 8 |
| | | Min. ≥4 g | 11 | 8 | 10 | 13 | 10 | 7 | 8 |
| Apparent Compressive Mod | | ≥0.6 g | 3.4 | 3.1 | 3.1 | 4.0 | 3.3 | 3.5 | 3.1 |
| Microbial Limits | | | | | | | | | |
| Total Aerobic plate count | G | ≤100 cfu per reservoir | 0.00 | NA | NA | NA | NA | N/A | |
| Anaerobes | | None Detected | ND | | | | | | |
| Pseudomonas aeruginosa | | None Detected | ND | | | | | | |
| Staphylococcus aureus | | None Detected | ND | | | | | | |
| Escherichia coli | | None Detected | ND | | | | | | |
| Salmonella sp. | | None Detected | ND | | | | | | |
| Clostridium perfringens | | None Detected | ND | | | | | | |

ND = None Detected          NA = Not Applicable          N/A = Not Analyzed

Table H *(continued)*

| Test – Cathode Reservoir | Method | Specification | Time in Months | | | | | | |
|---|---|---|---|---|---|---|---|---|---|
| | | | 0 | 3 | 6 | 9 | 12 | 18 | 24 |
| Preservative Assay | D | ≥3.0 mg/g | | NA | NA | NA | 4.9 | N/A | 4.9 |
| pH Hydrogel Surface | E | 4.0 – 6.0 | 4.6 | 4.7 | 4.7 | 4.7 | 4.6 | 4.7 | 4.7 |
| Probe Tack | F | Avg. ≥4 g | 8 | 6 | 7 | 8 | 7 | 8 | 7 |
| | | Min. ≥3 g | 7 | 6 | 6 | 8 | 7 | 7 | 6 |
| Apparent Compressive Mod | | ≥0.6 g | 3.3 | 3.0 | 2.9 | 4.1 | 3.4 | 3.9 | 3.4 |
| Microbial Limits | | | | | | | | | |
| Total Aerobic plate count | G | ≤100 cfu per reservoir | 0.00 | NA | NA | NA | NA | N/A | |
| Anaerobes | | None Detected | ND | | | | | | |
| Pseudomonas aeruginosa | | None Detected | ND | | | | | | |
| Staphylococcus aureus | | None Detected | ND | | | | | | |
| Escherichia coli | | None Detected | ND | | | | | | |
| Salmonella sp. | | None Detected | ND | | | | | | |
| Clostridium perfringens | | None Detected | ND | | | | | | |

ND = None Detected  NA = Not Applicable  N/A = Not Analyzed

EP 1 586 347 A1

24

Table H *(continued)*

| Test – Patch | Method | Specification | Time in Months | | | | | | |
|---|---|---|---|---|---|---|---|---|---|
| | | | 0 | 3 | 6 | 9 | 12 | 18 | 24 |
| **Physical** | | | | | | | | | |
| Probe Tack (Peripheral adhesive) | F | Avg. ≥150 g | 256 | 287 | 349 | 470 | 182 | 355 | 514 |
| | | Min. ≥50 g | 224 | 251 | 299 | 399 | 121 | 277 | 290 |
| Electrochemical/Electrical Properties | | | | | | | | | |
| Specific Capacity | | | | | | | | | |
| Anode | H | Avg. ≥6.7 mA-min/cm$^2$ | 18.4 | | | 18.7 | 19.0 | 17.9 | 18.5 |
| | | Min. ≥5.6 mA-min/cm$^2$ | 18.1 | | | 18.3 | 18.9 | 17.1 | 18.1 |
| Cathode | | ≥7.4 mA-min/cm$^2$ | 14.5 | | | 14.4 | 13.8 | 14.6 | 14.2 |
| Dielectric Leakage Current | I | Avg. ≤44.4 uA/in | 6.8 | 11.0 | 0.9 | 15.7 | 10.9 | 9.5 | 5.0 |
| | | Max. ≤55.5 uA/in | 13.9 | 13.4 | 1.6 | 25.0 | 19.4 | 15.0 | 5.9 |
| Patch Leakage Current | J | ≤0.62 uA @ 35 V | 0.00 | 0.00 | 0.00 | 0.00 | 0.00 | 0.00 | 0.00 |
| Patch Conductance | | | | | | | | | |
| **Trace Conductance** | | | | | | | | | |
| Anode | K | ≥0.001 (ohm)$^{-1}$ | 0.1597 | 0.1373 | 0.1268 | 0.1302 | 0.1212 | 0.1260 | 0.0931 |
| Cathode | | ≥0.001 (ohm)$^{-1}$ | 0.1587 | 0.1338 | 0.1240 | 0.1267 | 0.1171 | 0.1260 | 0.1160 |
| **Hydrogel/Electrode Conductivity** | | | | | | | | | |
| Anode | L | Avg. ≥0.0050 (ohm-cm)$^{-1}$ | 0.0063 | 0.0058 | 0.0064 | 0.0060 | 0.0058 | 0.0061 | 0.0073 |
| | | Min. ≥0.0042 (ohm-cm)$^{-1}$ | 0.0061 | 0.0042 | 0.0060 | 0.0053 | 0.0045 | 0.0057 | 0.0063 |
| Cathode | | Avg. ≥0.0031 (ohm-cm)$^{-1}$ | 0.0063 | 0.0058 | 0.0057 | 0.0061 | 0.0062 | 0.0064 | 0.0063 |
| | | Min. ≥0.0028 (ohm-cm)$^{-1}$ | 0.0062 | 0.0052 | 0.0049 | 0.0055 | 0.0046 | 0.0060 | 0.0057 |

| Test – Container Closure | Method | Specification | Time in Months | | | | | | |
|---|---|---|---|---|---|---|---|---|---|
| | | | 0 | 3 | 6 | 9 | 12 | 18 | 24 |
| Opening force | M | 1000 - 2400 g | 1636 | 1522 | 1674 | 1731 | 1568 | 1576 | 1589 |
| Burst strength | N | 6 - 18 psi | 10.3 | 10.7 | 12.5 | 11.8 | 11.2 | 10.7 | 10.5 |

ND = None Detected          NA = Not Applicable          N/A = Not Analyzed

EP 1 586 347 A1

Table I: Refrigerated Temperature (5°C) Stability Data          Lot Number 2
95.0 mg Lidocaine HCl/ 1.0 mg Epinephrine free base Iontophoretic patch

| Test – Anode Reservoir | Method | Specification | Time in Months | | | | | | |
|---|---|---|---|---|---|---|---|---|---|
| | | | 0 | 3 | 6 | 9 | 12 | 18 | 24 |
| **Drug Assay** | | | | | | | | | |
| Lidocaine HCl Assay | A | 85.5 - 104.5 mg/patch | 99.8 | 99.8 | | 98.7 | 97.0 | N/A | N/A |
| Epinephrine Assay | | 0.85 – 1.10 mg/patch | 1.02 | 1.02 | 1.03 | 1.03 | 1.02 | N/A | N/A |
| Lidocaine Degradants | | | | | | | | | |
| Individual Unidentified Degs | b | ≤200 ug/patch | ND | ND | ND | NA | ND | N/A | N/A |
| Total Lidocaine Degs | | ≤200 ug/patch | ND | ND | ND | ND | ND | N/A | N/A |
| Epinephrine Degradants | | | | | | | | | |
| Epinephrine Sulfonic Acid | C | ≤100 ug/patch | 3.6 | 5.6 | 5.8 | 6.4 | 7.3 | N/A | N/A |
| Adrenalone | | ≤10 ug/patch | ND | ND | ND | ND | ND | N/A | N/A |
| Individual Unidentified Degs | | ≤5 ug/patch | ND | ND | ND | ND | ND | N/A | N/A |
| Total Epinephrine Degs | | ≤150 ug/patch | 3.6 | 5.6 | 5.8 | 6.4 | 7.3 | N/A | N/A |
| Total Degradants | B C | ≤350 ug/patch | 3.6 | 5.6 | 5.8 | 6.4 | 7.3 | N/A | N/A |
| Preservative Assay | D | ≥3.0 mg/g | | NA | NA | NA | 5.0 | N/A | N/A |
| pH Hydrogel Surface | E | 3.7 - 4.5 | 3.9 | 4.1 | 4.1 | 4.2 | 4.1 | N/A | N/A |
| Probe Tack | F | Avg. ≥6 g | 18 | 13 | 10 | 11 | 11 | N/A | N/A |
| | | Min. ≥4 g | 13 | 9 | 10 | 8 | 9 | N/A | N/A |
| Apparent Compressive Mod | | ≥0.6 g | 3.4 | 3.1 | 3.2 | 3.4 | 3.1 | N/A | N/A |

EP 1 586 347 A1

Table I *(continued)*

| Test – Anode Reservoir | Method | Specification | 0 | 3 | 6 | Time in Months 9 | 12 | 18 | 24 |
|---|---|---|---|---|---|---|---|---|---|
| Microbial Limits | | | | | | | | | |
| Total Aerobic plate count | G | ≤100 cfu per reservoir | 0.00 | NA | NA | NA | NA | N/A | N/A |
| Anaerobes | | None Detected | ND | | | | | | |
| Pseudomonas aeruginosa | | None Detected | ND | | | | | | |
| Staphylococcus aureus | | None Detected | ND | | | | | | |
| Escherichia coli | | None Detected | ND | | | | | | |
| Salmonella sp. | | None Detected | ND | | | | | | |
| Clostridium perfringens | | None Detected | ND | | | | | | |

| Test – Cathode Reservoir | Method | Specification | 0 | 3 | 6 | Time in Months 9 | 12 | 18 | 24 |
|---|---|---|---|---|---|---|---|---|---|
| Preservative Assay | D | ≥3.0 mg/g | | NA | NA | NA | 4.9 | N/A | N/A |
| pH Hydrogel Surface | E | 4.0 – 6.0 | 4.7 | 4.7 | 4.7 | 4.7 | 4.7 | N/A | N/A |
| Probe Tack | F | Avg. ≥4 g | 9 | 7 | 7 | 7 | 6 | N/A | N/A |
| | | Min. ≥3 g | 7 | 6 | 7 | 6 | 6 | N/A | N/A |
| Apparent Compressive Mod | | ≥0.6 g | 3.3 | 3.1 | 3.2 | 3.5 | 3.5 | N/A | N/A |
| Microbial Limits | | | | | | | | | |
| Total Aerobic plate count | G | ≤100 cfu per reservoir | 0.09 | NA | NA | NA | NA | N/A | N/A |
| Anaerobes | | None Detected | ND | | | | | | |
| Pseudomonas aeruginosa | | None Detected | ND | | | | | | |
| Staphylococcus aureus | | None Detected | ND | | | | | | |
| Escherichia coli | | None Detected | ND | | | | | | |
| Salmonella sp. | | None Detected | ND | | | | | | |
| Clostridium perfringens | | None Detected | ND | | | | | | |

ND = None Detected        NA = Not Applicable        N/A = Not Analyzed

Table I *(continued)*

| Test – Patch | Method | Specification | 0 | 3 | 6 | 9 | 12 | 18 | 24 |
|---|---|---|---|---|---|---|---|---|---|
| **Physical** | | | | | | | | | |
| Probe Tack (Peripheral adhesive) | F | Avg. ≥150 g | 300 | 426 | 601 | 323 | 512 | N/A | N/A |
| | | Min. ≥50 g | 208 | 396 | 531 | 294 | 450 | N/A | N/A |
| **Electrochemical/Electrical Properties** | | | | | | | | | |
| **Specific Capacity** | | | | | | | | | |
| Anode | H | Avg. ≥6.7 mA-min/cm² | 21.2 | | | 20.0 | 21.1 | N/A | N/A |
| | | Min. ≥5.6 mA-min/cm² | 19.3 | | | 18.2 | 20.9 | N/A | N/A |
| Cathode | | ≥7.4 mA-min/cm² | 15.1 | | | 14.9 | 15.2 | N/A | N/A |
| Dielectric Leakage Current | I | Avg. ≤44.4 uA/in | 8.1 | 8.7 | 1.4 | 2.8 | 2.2 | N/A | N/A |
| | | Max. ≤55.5 uA/in | 17.4 | 13.7 | 1.9 | 3.9 | 2.8 | N/A | N/A |
| Patch Leakage Current | J | ≤0.62 uA @ 35 V | 0.00 | 0.00 | 0.00 | 0.00 | 0.00 | N/A | N/A |
| **Patch Conductance** | | | | | | | | | |
| **Trace Conductance** | | | | | | | | | |
| Anode | K | ≥0.001 (ohm)⁻¹ | 0.1948 | 0.1589 | 0.1561 | 0.1535 | 0.1535 | N/A | N/A |
| Cathode | | ≥0.001 (ohm)⁻¹ | 0.1939 | 0.1525 | 0.1591 | 0.1550 | 0.1536 | N/A | N/A |
| **Hydrogel/Electrode Conductivity** | | | | | | | | | |
| Anode | L | Avg. ≥0.0050 (ohm-cm)⁻¹ | 0.0061 | 0.0059 | 0.0059 | 0.0063 | 0.0064 | N/A | N/A |
| | | Min. ≥0.0042 (ohm-cm)⁻¹ | 0.0060 | 0.0058 | 0.0055 | 0.0057 | 0.0059 | N/A | N/A |
| Cathode | | Avg. ≥0.0031 (ohm-cm)⁻¹ | 0.0060 | 0.0058 | 0.0051 | 0.0050 | 0.0055 | N/A | N/A |
| | | Min. ≥0.0028 (ohm-cm)⁻¹ | 0.0059 | 0.0046 | 0.0040 | 0.0028 | 0.0052 | N/A | N/A |

| Test – Container Closure | Method | Specification | 0 | 3 | 6 | 9 | 12 | 18 | 24 |
|---|---|---|---|---|---|---|---|---|---|
| Opening force | M | 1000 - 2400 g | 1642 | 1705 | 1434 | 1725 | 1463 | N/A | N/A |
| Burst strength | N | 6 - 18 psi | 10.7 | 10.3 | 11.9 | 11.7 | 13.4 | N/A | N/A |

ND = None Detected    NA = Not Applicable    N/A = Not Analyzed

EP 1 586 347 A1

**Table J: Refrigerated Temperature (5°C) Stability Data**      Lot Number 3

**95.0 mg Lidocaine HCl/ 1.0 mg Epinephrine free base Iontophoretic patch**

| Test – Anode Reservoir | Method | Specification | Time in Months | | | | | | |
|---|---|---|---|---|---|---|---|---|---|
| | | | 0 | 3 | 6 | 9 | 12 | 18 | 24 |
| Lidocaine HCl Assay | A | 85.5 - 104.5 mg/patch | 99.9 | 99.2 | | 98.5 | 99.2 | 97.7 | 95.7 |
| Epinephrine Assay | A | 0.85 – 1.10 mg/patch | 1.04 | 1.03 | 1.03 | 1.02 | 1.02 | 1.01 | 1.02 |
| Lidocaine Degradants | | | | | | | | | |
| Individual Unidentified Degs | B | ≤200 ug/patch | ND | ND | ND | N/A | ND | ND | ND |
| Total Lidocaine Degs | B | ≤200 ug/patch | ND | ND | ND | N/A | ND | ND | ND |
| Epinephrine Degradants | | | | | | | | | |
| Epinephrine Sulfonic Acid | C | ≤100 ug/patch | 3.1 | 5.6 | 5.3 | 6.4 | 6.8 | 6.6 | 6.5 |
| Adrenalone | C | ≤10 ug/patch | ND | ND | ND | ND | ND | ND | ND |
| Individual Unidentified Degs | C | ≤5 ug/patch | ND | ND | ND | ND | ND | ND | ND |
| Total Epinephrine Degs | C | ≤150 ug/patch | ND | ND | ND | ND | ND | ND | ND |
| Total Degradants | B C | ≤350 ug/patch | 3.1 | 5.6 | 5.3 | 6.4 | 6.8 | 6.6 | 6.5 |
| Preservative Assay | D | ≥3.0 mg/g | | NA | NA | NA | 5.1 | N/A | 5.1 |
| pH Hydrogel Surface | E | 3.7 - 4.5 | 4.1 | 4.1 | 4.1 | 4.1 | 4.2 | 4.1 | 4.2 |
| Probe Tack | F | Avg. ≥6 g | 15 | 16 | 11 | 12 | 11 | 11 | 11 |
| | F | Min.. ≥4 g | 13 | 11 | 9 | 10 | 10 | 8 | 9 |
| Apparent Compressive Mod | | ≥0.6 g | 3.4 | 2.9 | 2.8 | 3.9 | 3.2 | 3.2 | 3.1 |
| Microbial Limits | | | | | | | | | |
| Total Aerobic plate count | G | ≤100 cfu per reservoir | 0.00 | NA | NA | NA | NA | N/A | |
| Anaerobes | | None Detected | ND | | | | | | |
| Pseudomonas aeruginosa | | None Detected | ND | | | | | | |
| Staphylococcus aureus | | None Detected | ND | | | | | | |
| Escherichia coli | | None Detected | ND | | | | | | |
| Salmonella sp. | | None Detected | ND | | | | | | |
| Clostridium perfringens | | None Detected | ND | | | | | | |

ND = None Detected      NA = Not Applicable      N/A = Not Analyzed

Table J *(continued)*

| Test – Cathode Reservoir | Method | Specification | Time in Months | | | | | | |
|---|---|---|---|---|---|---|---|---|---|
| | | | 0 | 3 | 6 | 9 | 12 | 18 | 24 |
| Preservative Assay | D | ≥3.0 mg/g | | NA | NA | NA | 5.0 | N/A | 5.0 |
| pH Hydrogel Surface | E | 4.0 – 6.0 | 4.6 | 4.7 | 4.7 | 4.7 | 4.7 | 4.8 | 4.7 |
| Probe Tack | F | Avg. ≥4 g | 9 | 11 | 8 | 7 | 8 | 9 | 8 |
| | | Min. ≥3 g | 8 | 7 | 7 | 6 | 7 | 8 | 7 |
| Apparent Compressive Mod | | ≥0.6 g | 3.4 | 2.8 | 2.9 | 4.0 | 3.5 | 3.3 | 3.2 |
| Microbial Limits | | | | | | | | | |
| Total Aerobic plate count | G | ≤100 cfu per reservoir | 0.00 | NA | NA | NA | NA | N/A | |
| Anaerobes | | None Detected | ND | | | | | | |
| Pseudomonas aeruginosa | | None Detected | ND | | | | | | |
| Staphylococcus aureus | | None Detected | ND | | | | | | |
| Escherichia coli | | None Detected | ND | | | | | | |
| Salmonella sp. | | None Detected | ND | | | | | | |
| Clostridium perfringens | | None Detected | ND | | | | | | |

ND = None Detected          NA = Not Applicable          N/A = Not Analyzed

Table J *(continued)*

| Test – Patch | Method | Specification | Time in Months | | | | | | |
|---|---|---|---|---|---|---|---|---|---|
| | | | 0 | 3 | 6 | 9 | 12 | 18 | 24 |
| **Physical** | | | | | | | | | |
| Probe Tack (Peripheral adhesive) | F | Avg. ≥150 g | 342 | 474 | 444 | 747 | 378 | 143 | 366 |
| | | Min. ≥50 g | 305 | 339 | 219 | 687 | 336 | 122 | 300 |
| **Electrochemical/Electrical Properties** | | | | | | | | | |
| **Specific Capacity** | H | | | | | | | | |
| Anode | | Avg. ≥6.7 mA-min/cm² | 17.9 | | | 17.4 | 17.0 | 17.1 | 18.0 |
| | | Min. ≥5.6 mA-min/cm² | 16.8 | | | 16.1 | 16.7 | 17.0 | 17.2 |
| Cathode | | ≥7.4 mA-min/cm² | 14.8 | | | 14.4 | 14.7 | 14.9 | 14.5 |
| Dielectric Leakage Current | I | Avg. ≤44.4 uA/in | 4.7 | 6.0 | 1.2 | 11.1 | 2.6 | 11.4 | 11.2 |
| | | Max. ≤55.5 uA/in | 8.9 | 6.5 | 1.5 | 12.7 | 3.0 | 17.5 | 15.8 |
| Patch Leakage Current | J | ≤0.62 uA @ 35 V | 0.00 | | 0.00 | 0.00 | 0.00 | 0.00 | 0.00 |
| **Patch Conductance** | | | | | | | | | |
| **Trace Conductance** | K | | | | | | | | |
| Anode | | ≥0.001 $(ohm)^{-1}$ | 0.1556 | 0.1200 | 0.1116 | 0.1333 | 0.1296 | 0.1125 | 0.1299 |
| Cathode | | ≥0.001 $(ohm)^{-1}$ | 0.1540 | 0.0519 | 0.1131 | 0.1299 | 0.1307 | 0.1137 | 0.1300 |
| **Hydrogel/Electrode Conductivity** | L | | | | | | | | |
| Anode | | Avg. ≥0.0050 $(ohm\text{-}cm)^{-1}$ | 0.0062 | 0.0061 | 0.0060 | 0.0060 | 0.0059 | 0.0065 | 0.0066 |
| | | Min. ≥0.0042 $(ohm\text{-}cm)^{-1}$ | 0.0061 | 0.0052 | 0.0053 | 0.0056 | 0.0053 | 0.0064 | 0.0064 |
| Cathode | | Avg. ≥0.0031 $(ohm\text{-}cm)^{-1}$ | 0.0061 | 0.0060 | 0.0054 | 0.0055 | 0.0057 | 0.0067 | 0.0064 |
| | | Min. ≥0.0028 $(ohm\text{-}cm)^{-1}$ | 0.0059 | 0.0046 | 0.0048 | 0.0048 | 0.0051 | 0.0062 | 0.0058 |

| Test – Container Closure | Method | Specification | Time in Months | | | | | | |
|---|---|---|---|---|---|---|---|---|---|
| | | | 0 | 3 | 6 | 9 | 12 | 18 | 24 |
| Opening force | M | 1000 - 2400 g | 1551 | 1439 | 1656 | 1610 | 1625 | 1536 | 1547 |
| Burst strength | N | 6 - 18 psi | 9.3 | 9.3 | 11.8 | 11.7 | 12.4 | 10.3 | 9.4 |

NA = Not Applicable    N/A = Not Analyzed

ND = None Detected

## B. Stability Data 25°C/60% RH

Long-term stability data at 25°C/60% RH for lots 1, 2 and 3 are contained within Tables K, L and M, respectively. All data are within the proposed specifications at all time points through 24 months. The relative humidities along with the temperatures are controlled to determine if the package would be compromised during stability testing. For the foil-laminate packaging used herein, however, there was no adverse affect on the packaging at all testing conditions, irrespective of the humidity or temperature, for all time points.

Table K: Room Temperature (25°C/60% RH) Stability Data      Lot Number 1
**95.0 mg Lidocaine HCl/ 1.0 mg Epinephrine free base Iontophoretic patch**

| Test – Anode Reservoir | Method | Specification | Time in Months | | | | | | |
|---|---|---|---|---|---|---|---|---|---|
| | | | 0 | 3 | 6 | 9 | 12 | 18 | 24 |
| **Drug Assay** | | | | | | | | | |
| Lidocaine HCl Assay | A | 85.5 – 104.5 mg/patch | 102.3 | 100.7 | 100.7 | 99.2 | 96.6 | 97.7 | 95.8 |
| Epinephrine Assay | | 0.85 – 1.10 mg/patch | 1.06 | 1.02 | 1.02 | 1.00 | 0.99 | 0.98 | 0.97 |
| Lidocaine Degradants | | | | | | | | | |
| Individual Unidentified Degs | B | ≤200 ug/patch | ND | ND | ND | N/A | ND | ND | ND |
| Total Lidocaine Degs | | ≤200 ug/patch | ND | ND | ND | N/A | ND | ND | ND |
| Epinephrine Degradants | | | | | | | | | |
| Epinephrine Sulfonic Acid | C | ≤100 ug/patch | 4.4 | 11.5 | 16.6 | 22.4 | 29.0 | 36.7 | 42.1 |
| Adrenalone | | ≤10 ug/patch | ND | ND | ND | N/A | N/A | N/A | 0.7 |
| Individual Unidentified Degs | | ≤5 ug/patch | ND | ND | ND | ND | ND | ND | ND |
| Total Epinephrine Degs | | ≤150 ug/patch | 4.4 | 11.5 | 16.6 | 22.4 | 29.0 | 36.7 | 42.8 |
| **Total Degradants** | B C | ≤350 ug/patch | 4.4 | 11.5 | 16.6 | 22.6 | 29.4 | 36.9 | 42.8 |
| Preservative Assay | D | ≥3.0 mg/g | | NA | NA | NA | 4.6 | 4.4 | 4.4 |
| pH Hydrogel Surface | E | 3.7 - 4.5 | 4.0 | 4.1 | 4.1 | 4.2 | 4.2 | 4.1 | 4.2 |
| Probe Tack | F | Avg. ≥6 g | 13 | 10 | 11 | 13 | 13 | 11 | 8.7 |
| | | Min. ≥4 g | 11 | 9 | 9 | 11 | 10 | 10 | 8 |
| Apparent Compressive Mod | | ≥0.6 g | 3.4 | 3 | 3.1 | 4.1 | 3.7 | 3.5 | 3.1 |
| Microbial Limits | | | | | | | | | |
| Total Aerobic plate count | G | ≤100 cfu per reservoir | 0.00 | NA | NA | NA | 0.00 | N/A | |
| Anaerobes | | None Detected | ND | | | | ND | | |
| Pseudomonas aeruginosa | | None Detected | ND | | | | ND | | |
| Staphylococcus aureus | | None Detected | ND | | | | ND | | |
| Escherichia coli | | None Detected | ND | | | | ND | | |
| Salmonella sp. | | None Detected | ND | | | | ND | | |
| Clostridium perfringens | | None Detected | ND | | | | ND | | |

ND = None Detected      NA = Not Applicable      N/A = Not Analyzed

Table K *(continued)*

| Test – Cathode Reservoir | Method | Specification | Time in Months | | | | | | |
|---|---|---|---|---|---|---|---|---|---|
| | | | 0 | 3 | 6 | 9 | 12 | 18 | 24 |
| Preservative Assay | D | ≥3.0 mg/g | | NA | NA | NA | 3.7 | 3.3 | 3.2 |
| pH Hydrogel Surface | E | 4.0 – 6.0 | 4.6 | 4.6 | 4.6 | 4.6 | 4.6 | 4.6 | 4.6 |
| Probe Tack | F | Avg. ≥4 g | 8 | 7 | 8 | 8 | 9 | 10 | 9 |
| | | Min. ≥3 g | 7 | 7 | 7 | 7 | 7 | 9 | 8 |
| Apparent Compressive Mod | | ≥0.6 g | 3.3 | 3.1 | 3.1 | 4.3 | 3.7 | 3.8 | 3.1 |
| Microbial Limits | | | | | | | | | |
| Total Aerobic plate count | G | ≤100 cfu per reservoir | 0.00 | NA | NA | NA | 0.00 | N/A | |
| Anaerobes | | None Detected | ND | | | | ND | | |
| Pseudomonas aeruginosa | | None Detected | ND | | | | ND | | |
| Staphylococcus aureus | | None Detected | ND | | | | ND | | |
| Escherichia coli | | None Detected | ND | | | | ND | | |
| Salmonella sp. | | None Detected | ND | | | | ND | | |
| Clostridium perfringens | | None Detected | ND | | | | ND | | |

ND = None Detected   NA = Not Applicable   N/A = Not Analyzed

EP 1 586 347 A1

Table K *(continued)*

| Test – Patch | Method | Specification | Time in Months | | | | | | |
|---|---|---|---|---|---|---|---|---|---|
| | | | 0 | 3 | 6 | 9 | 12 | 18 | 24 |
| **Physical** | | | | | | | | | |
| Probe Tack (Peripheral adhesive) | F | Avg. ≥150 g | 256 | 267 | 380 | 297 | 312 | 300 | 681 |
| | | Min. ≥50 g | 224 | 178 | 321 | 251 | 198 | 249 | 599 |
| Electrochemical/Electrical Properties | | | | | | | | | |
| Specific Capacity | | | | | | | | | |
| Anode | H | Avg. ≥6.7 mA-min/cm$^2$ | 18.4 | | | 17.1 | 16.8 | 16.7 | 17.4 |
| | | Min. ≥5.6 mA-min/cm$^2$ | 18.1 | | | 15.8 | 16.6 | 16.2 | 17.1 |
| Cathode | | ≥7.4 mA-min/cm$^2$ | 14.5 | | | 13.8 | 13.9 | 13.8 | 13.9 |
| Dielectric Leakage Current | I | Avg. ≤44.4 uA/in | 6.8 | 5.7 | 1.7 | 7.0 | 10.4 | 4.2 | 2.5 |
| | | Max. ≤55.5 uA/in | 13.9 | 7.4 | 2 | 7.9 | 11.7 | 5.8 | 4.0 |
| Patch Leakage Current | J | ≤0.62 uA @ 35 V | 0.00 | 0.00 | 0.00 | 0.00 | 0.00 | 0.00 | 0.00 |
| Patch Conductance | | | | | | | | | |
| **Trace Conductance** | | | | | | | | | |
| Anode | K | ≥0.001 (ohm)$^{-1}$ | 0.1597 | 0.0835 | 0.0480 | 0.0513 | 0.0433 | 0.0203 | 0.0139 |
| Cathode | | ≥0.001 (ohm)$^{-1}$ | 0.1587 | 0.0865 | 0.0540 | 0.0469 | 0.0474 | 0.0245 | 0.0167 |
| **Hydrogel/Electrode Conductivity** | | | | | | | | | |
| Anode | L | Avg. ≥0.0050 (ohm-cm)$^{-1}$ | 0.0063 | 0.0058 | 0.0056 | 0.0061 | 0.0056 | 0.0065 | 0.0066 |
| | | Min. ≥0.0042 (ohm-cm)$^{-1}$ | 0.0061 | 0.0052 | 0.0046 | 0.0049 | 0.0039 | 0.0061 | 0.0059 |
| Cathode | | Avg. ≥0.0031 (ohm-cm)$^{-1}$ | 0.0063 | 0.0054 | 0.0052 | 0.0060 | 0.0052 | 0.0062 | 0.0058 |
| | | Min. ≥0.0028 (ohm-cm)$^{-1}$ | 0.0062 | 0.0050 | 0.0042 | 0.0052 | 0.0034 | 0.0055 | 0.0027 |

| Test – Container Closure | Method | Specification | Time in Months | | | | | | |
|---|---|---|---|---|---|---|---|---|---|
| | | | 0 | 3 | 6 | 9 | 12 | 18 | 24 |
| Opening force | M | 1000 - 2400 g | 1636 | 1522 | 1510 | 1504 | 1674 | 1440 | 1806 |
| Burst strength | N | 6 - 18 psi | 10.3 | 10.1 | 12.4 | 12.9 | 12.6 | 9.7 | 10.8 |

ND = None Detected          NA = Not Applicable          N/A = Not Analyzed

34

EP 1 586 347 A1

Table L: Room Temperature (25°C/60% RH) Stability Data        Lot Number 2
95.0 mg Lidocaine HCl/ 1.0 mg Epinephrine free base Iontophoretic patch

| Test – Anode Reservoir | Method | Specification | 0 | 3 | 6 | 9 | 12 | 18 | 24 |
|---|---|---|---|---|---|---|---|---|---|
| **Drug Assay** | | | | | | | | | |
| Lidocaine HCl Assay | A | 85.5 - 104.5 mg/patch | 99.8 | 98.4 | 97.4 | 97.0 | 97.4 | 96.6 | 95.1 |
| Epinephrine Assay | | 0.85 – 1.10 mg/patch | 1.02 | 1.01 | 1.01 | 0.99 | 0.98 | 0.98 | 0.96 |
| **Lidocaine Degradants** | | | | | | | | | |
| Individual Unidentified Degs | B | ≤200 ug/patch | ND | ND | ND | NA | ND | ND | ND |
| Total Lidocaine Degs | | ≤200 ug/patch | ND | ND | ND | ND | ND | ND | ND |
| **Epinephrine Degradants** | | | | | | | | | |
| Epinephrine Sulfonic Acid | C | ≤100 ug/patch | 3.6 | 11.5 | 16.7 | 22.2 | 28.3 | 36.7 | 43.3 |
| Adrenalone | | ≤10 ug/patch | ND | ND | ND | N/A | N/A | N/A | 0.6 |
| Individual Unidentified Degs | | ≤5 ug/patch | ND | ND | ND | ND | ND | ND | ND |
| Total Epinephrine Degs | | ≤150 ug/patch | 3.6 | 11.5 | 16.7 | 22.2 | 28.3 | 36.7 | 43.9 |
| Total Degradants | B C | ≤350 ug/patch | 3.6 | 11.5 | 16.7 | 22.2 | 28.3 | 36.7 | 43.9 |
| Preservative Assay | D | ≥3.0 mg/g | | NA | NA | NA | 4.7 | 4.9 | 4.6 |
| PH Hydrogel Surface | E | 3.7 - 4.5 | 3.9 | 4.1 | 4.1 | 4.2 | 4.2 | 4.1 | 4.1 |
| Probe Tack | F | Avg. ≥6 g | 18 | 9 | 10 | 10 | 12 | 10 | 9 |
| | | Min. ≥4 g | 13 | 9 | 9 | 10 | 10 | 9 | 8 |
| Apparent Compressive Mod | | ≥0.6 g | 3.4 | 3.2 | 3.3 | 3.7 | 3.0 | 3.2 | 3.0 |
| **Microbial Limits** | | | | | | | | | |
| Total Aerobic plate count | G | ≤100 cfu per reservoir | 0.00 | NA | NA | NA | 0.00 | N/A | |
| Anaerobes | | None Detected | ND | | | | ND | | |
| Pseudomonas aeruginosa | | None Detected | ND | | | | ND | | |
| Staphylococcus aureus | | None Detected | ND | | | | ND | | |
| Escherichia coli | | None Detected | ND | | | | ND | | |
| Salmonella sp. | | None Detected | ND | | | | ND | | |
| Clostridium perfringens | | None Detected | ND | | | | ND | | |

ND = None Detected        NA = Not Applicable        N/A = Not Analyzed

EP 1 586 347 A1

Table L *(continued)*

| Test – Cathode Reservoir | Method | Specification | Time in Months | | | | | | |
|---|---|---|---|---|---|---|---|---|---|
| | | | 0 | 3 | 6 | 9 | 12 | 18 | 24 |
| Preservative Assay | D | ≥3.0 mg/g | | NA | NA | NA | 3.7 | 3.5 | 3.3 |
| PH Hydrogel Surface | E | 4.0 – 6.0 | 4.7 | 4.7 | 4.7 | 4.6 | 4.7 | 4.7 | 4.6 |
| Probe Tack | F | Avg. ≥4 g | 9 | 6 | 8 | 7 | 9 | 10 | 8 |
| | | Min. ≥3 g | 7 | 6 | 8 | 6 | 8 | 10 | 7 |
| Apparent Compressive Mod | | ≥0.6 g | 3.3 | 3.3 | 3.3 | 3.8 | 3.2 | 3.5 | 3.4 |
| Microbial Limits | | | | | | | | | |
| Total Aerobic plate count | G | ≤100 cfu per reservoir | 0.09 | NA | NA | NA | 0.00 | N/A | |
| Anaerobes | | None Detected | ND | | | | ND | | |
| Pseudomonas aeruginosa | | None Detected | ND | | | | ND | | |
| Staphylococcus aureus | | None Detected | ND | | | | ND | | |
| Escherichia coli | | None Detected | ND | | | | ND | | |
| Salmonella sp. | | None Detected | ND | | | | ND | | |
| Clostridium perfringens | | None Detected | ND | | | | ND | | |

ND = None Detected          NA = Not Applicable          N/A = Not Analyzed

Table L *(continued)*

| Test – Patch | Method | Specification | Time in Months | | | | | | |
|---|---|---|---|---|---|---|---|---|---|
| | | | 0 | 3 | 6 | 9 | 12 | 18 | 24 |
| Physical | | | | | | | | | |
| Probe Tack (Peripheral adhesive) | F | Avg. ≥150 g | 300 | 349 | 521 | 525 | 504 | 507 | 407 |
| | | Min. ≥50 g | 208 | 251 | 330 | 206 | 268 | 290 | 225 |
| Electrochemical/Electrical Properties | | | | | | | | | |
| Specific Capacity | | | | | | | | | |
| Anode | H | Avg. ≥6.7 mA-min/cm² | 21.2 | | | 20.8 | 18.5 | 19.6 | 19.2 |
| | | Min. ≥5.6 mA-min/cm² | 19.3 | | | 20.3 | 18.2 | 16.7 | 18.0 |
| Cathode | | ≥7.4 mA-min/cm² | 15.1 | | | 14.9 | 14.1 | 14.7 | 14.2 |
| Dielectric Leakage Current | I | Avg. ≤44.4 uA/in | 8.1 | 7.4 | 2.4 | 3.7 | 2.2 | 8.8 | 11.2 |
| | | Max. ≤55.5 uA/in | 17.4 | 9.8 | 3.1 | 4.5 | 2.6 | 11.1 | 15.8 |
| Patch Leakage Current | J | ≤0.62 uA @ 35 V | 0.00 | 0.00 | 0.00 | 0.00 | 0.00 | 0.00 | 0.00 |
| Patch Conductance | | | | | | | | | |
| Trace Conductance | | | | | | | | | |
| Anode | K | ≥0.001 (ohm)⁻¹ | 0.1948 | 0.0838 | 0.0782 | 0.0604 | 0.0501 | 0.0271 | 0.0259 |
| Cathode | | ≥0.001 (ohm)⁻¹ | 0.1939 | 0.0684 | 0.0809 | 0.0622 | 0.0528 | 0.0287 | 0.0238 |
| Hydrogel/Electrode Conductivity | | | | | | | | | |
| Anode | L | Avg. ≥0.0050 (ohm-cm)⁻¹ | 0.0061 | 0.0060 | 0.0058 | 0.0066 | 0.0057 | 0.0066 | 0.0063 |
| | | Min. ≥0.0042 (ohm-cm)⁻¹ | 0.0060 | 0.0037 | 0.0051 | 0.0059 | 0.0049 | 0.0061 | 0.0053 |
| Cathode | | Avg. ≥0.0031 (ohm-cm)⁻¹ | 0.0060 | 0.0060 | 0.0048 | 0.0056 | 0.0051 | 0.0053 | 0.0064 |
| | | Min. ≥0.0028 (ohm-cm)⁻¹ | 0.0059 | 0.0047 | 0.0037 | 0.0047 | 0.0034 | 0.0030 | 0.0059 |

| Test – Container Closure | Method | Specification | Time in Months | | | | | | |
|---|---|---|---|---|---|---|---|---|---|
| | | | 0 | 3 | 6 | 9 | 12 | 18 | 24 |
| Opening force | M | 1000 - 2400 g | 1642 | 1462 | 1515 | 1503 | 1486 | 1428 | 1657 |
| Burst strength | N | 6 - 18 psi | 10.7 | 11.3 | 13.4 | 12.3 | 12.4 | 13.0 | 9.3 |

ND = None Detected          NA = Not Applicable          N/A = Not Analyzed

Table M: Room Temperature (25°C/60% RH) Stability Data Lot Number 3

Product Description: 95.0 mg Lidocaine HCl/ 1.0 mg Epinephrine free base Iontophoretic patch

| Test – Anode Reservoir | Method | Specification | 0 | 3 | 6 | 9 | 12 | 18 | 24 |
|---|---|---|---|---|---|---|---|---|---|
| Lidocaine HCl Assay | A | 85.5 - 104.5 mg/patch | 99.9 | 100.0 | 97.9 | 97.5 | 95.7 | 96.5 | 95.2 |
| Epinephrine Assay | | 0.85 – 1.10 mg/patch | 1.04 | 1.01 | 1.00 | 0.99 | 0.98 | 0.98 | 0.96 |
| Lidocaine Degradants | | | | | | | | | |
| Individual Unidentified Degs | B | ≤200 ug/patch | ND | ND | ND | N/A | ND | ND | ND |
| Total Lidocaine Degs | | ≤200 ug/patch | ND | ND | ND | N/A | ND | ND | ND |
| Epinephrine Degradants | | | | | | | | | |
| Epinephrine Sulfonic Acid | C | ≤100 ug/patch | 3.1 | 11.4 | 16.4 | 22.6 | 31.4 | 35.7 | 40.0 |
| Adrenalone | | ≤10 ug/patch | ND | ND | ND | N/A | N/A | ND | 2.0 |
| Individual Unidentified Degs | | ≤5 ug/patch | ND | ND | ND | ND | ND | ND | ND |
| Total Epinephrine Degs | | ≤150 ug/patch | 3.1 | 11.4 | 16.4 | 22.6 | 31.4 | 35.7 | 42.0 |
| Total Degradants | B C | ≤350 ug/patch | 3.1 | 11.4 | 16.4 | 22.6 | 31.4 | 35.7 | 42.0 |
| Preservative Assay | D | ≥3.0 mg/g | 5.5 | NA | NA | NA | 4.6 | 4.6 | 4.5 |
| pH Hydrogel Surface | E | 3.7 - 4.5 | 4.1 | 4.2 | 4.1 | 4.2 | 4.2 | 4.1 | 4.2 |
| Probe Tack | F | Avg. ≥6 g | 15 | 11 | 11 | 12 | 11 | 10 | 10 |
| | | Min.. ≥4 g | 13 | 10 | 9 | 10 | 10 | 8 | 8 |
| Apparent Compressive Mod | | ≥0.6 g | 3.4 | 3.0 | 2.7 | 3.7 | 3.3 | 3.4 | 3.1 |
| Microbial Limits | | | | | | | | | |
| Total Aerobic plate count | G | ≤100 cfu per reservoir | 0.00 | NA | NA | NA | 0.00 | N/A | |
| Anaerobes | | None Detected | ND | | | | ND | | |
| Pseudomonas aeruginosa | | None Detected | ND | | | | ND | | |
| Staphylococcus aureus | | None Detected | ND | | | | ND | | |
| Escherichia coli | | None Detected | ND | | | | ND | | |
| Salmonella sp. | | None Detected | ND | | | | ND | | |
| Clostridium perfringens | | None Detected | ND | | | | ND | | |

ND = None Detected       NA = Not Applicable       N/A = Not Analyzed

EP 1 586 347 A1

Table M *(continued)*

| Test – Cathode Reservoir | Method | Specification | Time in Months | | | | | | |
|---|---|---|---|---|---|---|---|---|---|
| | | | 0 | 3 | 6 | 9 | 12 | 18 | 24 |
| Preservative Assay | D | ≥3.0 mg/g | | NA | NA | NA | 3.6 | 3.5 | 3.3 |
| pH Hydrogel Surface | E | 4.0 – 6.0 | 4.6 | 4.7 | 4.6 | 4.6 | 4.6 | 4.6 | 4.5 |
| Probe Tack | F | Avg. ≥4 g | 9 | 8 | 8 | 8 | 8 | 11 | 10 |
| | | Min. ≥3 g | 8 | 7 | 7 | 8 | 7 | 8 | 8 |
| Apparent Compressive Mod | | ≥0.6 g | 3.4 | 3.0 | 2.8 | 3.6 | 3.2 | 3.7 | 3.3 |
| Microbial Limits | | | | | | | | | |
| Total Aerobic plate count | G | ≤100 cfu per reservoir | 0.00 | NA | NA | NA | 0.00 | N/A | |
| Anaerobes | | None Detected | ND | | | | ND | | |
| Pseudomonas aeruginosa | | None Detected | ND | | | | ND | | |
| Staphylococcus aureus | | None Detected | ND | | | | ND | | |
| Escherichia coli | | None Detected | ND | | | | ND | | |
| Salmonella sp. | | None Detected | ND | | | | ND | | |
| Clostridium perfringens | | None Detected | ND | | | | ND | | |

ND = None Detected    NA = Not Applicable    N/A = Not Analyzed

Table M *(continued)*

| Test – Patch | Method | Specification | Time in Months | | | | | | |
|---|---|---|---|---|---|---|---|---|---|
| | | | 0 | 3 | 6 | 9 | 12 | 18 | 24 |
| **Physical** | | | | | | | | | |
| Probe Tack (Peripheral adhesive) | F | Avg. ≥150 g | 342 | 618 | 290 | 656 | 433 | 599 | 438 |
| | | Min. ≥50 g | 305 | 433 | 261 | 512 | 166 | 568 | 333 |
| **Electrochemical/Electrical Properties** | | | | | | | | | |
| **Specific Capacity** | | | | | | | | | |
| Anode | H | Avg. ≥6.7 mA-min/cm² | 17.9 | | | 16.7 | 16.6 | 16.5 | 16.5 |
| | | Min. ≥5.6 mA-min/cm² | 16.8 | | | 15.6 | 16.2 | 16.1 | 16.0 |
| Cathode | | ≥7.4 mA-min/cm² | 14.8 | | | 13.8 | 14.0 | 14.1 | 14.4 |
| Dielectric Leakage Current | I | Avg. ≤44.4 uA/in | 4.7 | 7.2 | 2.5 | 12.4 | 6.6 | 7.0 | 6.8 |
| | | Max. ≤55.5 uA/in | 8.9 | 9.0 | 3.3 | 16.8 | 8.7 | 13.7 | 10.3 |
| Patch Leakage Current | J | ≤0.62 uA @ 35 V | 0.00 | 0.00 | 0.00 | 0.00 | 0.00 | 0.00 | 0.00 |
| **Patch Conductance** | | | | | | | | | |
| **Trace Conductance** | | | | | | | | | |
| Anode | K | ≥0.001 (ohm)⁻¹ | 0.1556 | 0.0738 | 0.0688 | 0.0516 | 0.0313 | 0.0253 | 0.0115 |
| Cathode | | ≥0.001 (ohm)⁻¹ | 0.1540 | 0.0814 | 0.0705 | 0.0498 | 0.0349 | 0.0281 | 0.0147 |
| **Hydrogel/Electrode Conductivity** | | | | | | | | | |
| Anode | L | Avg. ≥0.0050 (ohm-cm)⁻¹ | 0.0062 | 0.0059 | 0.0059 | 0.0060 | 0.0063 | 0.0064 | 0.0066 |
| | | Min. ≥0.0042 (ohm-cm)⁻¹ | 0.0061 | 0.0045 | 0.0052 | 0.0054 | 0.0056 | 0.0060 | 0.0063 |
| Cathode | | Avg. ≥0.0031 (ohm-cm)⁻¹ | 0.0061 | 0.0057 | 0.0050 | 0.0054 | 0.0054 | 0.0055 | 0.0066 |
| | | Min. ≥0.0028 (ohm-cm)⁻¹ | 0.0059 | 0.0045 | 0.0047 | 0.0043 | 0.0047 | 0.0048 | 0.0065 |

| Test – Container Closure | Method | Specification | Time in Months | | | | | | |
|---|---|---|---|---|---|---|---|---|---|
| | | | 0 | 3 | 6 | 9 | 12 | 18 | 24 |
| Opening force | M | 1000 - 2400 g | 1551 | 1447 | 1700 | 1515 | 1494 | 1605 | 1541 |
| Burst strength | N | 6 - 18 psi | 9.3 | 9.8 | 12.6 | 12.3 | 11.8 | 12.2 | 12.5 |

NA = Not Applicable    N/A = Not Analyzed

ND = None Detected

## Epinephrine Potency and Degradants Assay

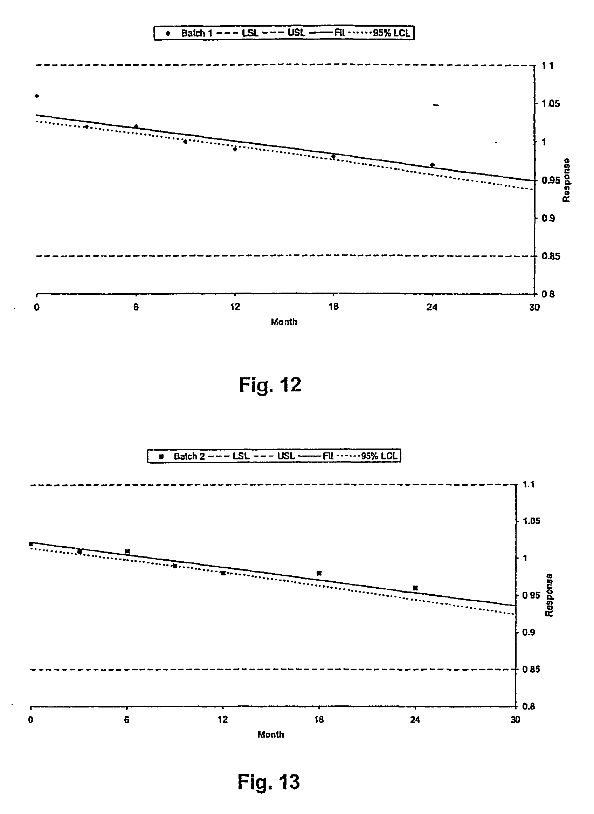

Epinephrine Potency Assay data at 25°C/60% RH for lots 1, 2 and 3 are contained in Tables K, L and M, above, respectively. Epinephrine linear regression data together with determination of the 95% lower confidence limit for lots 1, 2 and 3 are contained in Figures 12, 13 and 14, respectively. The equation of the line for each of the three lots is as follows:

Figure 12 - % Label claim for Lot 1 = 113.8 - 0.286 Time (months)
Figure 13 - % Label claim for Lot 2 =102.3 - 0.286 Time (months)

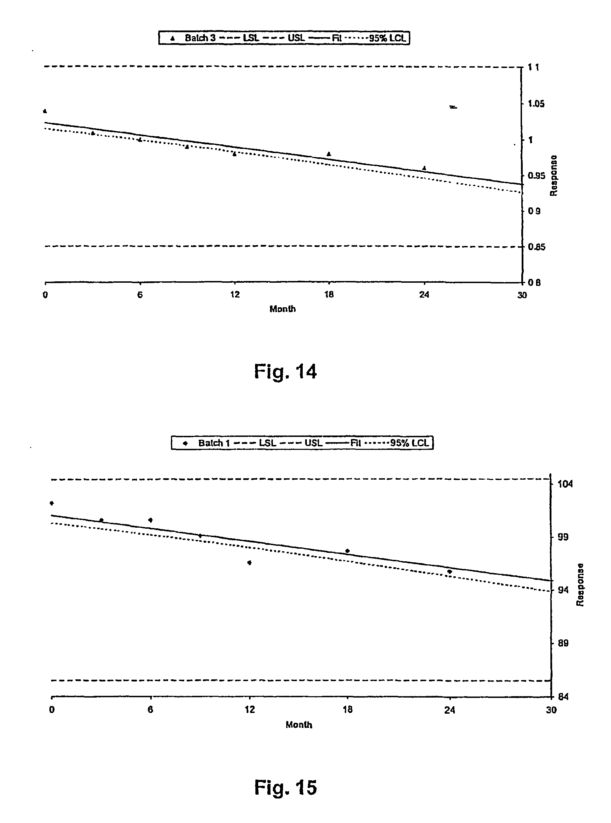

Figure 14 - % Label claim for Lot 3 =102.4 - 0.286 Time (months)

For ease of review, the epinephrine potency data used to generate Figures 12, 13 and 14 are included below as Tables N, O and P, respectively. Data are presented in Tables N, O and P as mg/patch and percent label claim. Data are provided as percent label claim. By projected linear regression, a shelf life of greater than 52 months is obtained.

TABLE N -

| Epinephrine Data at 25°C/60% RH in mg/patch and the Percent Label Claim | | |
| --- | --- | --- |
| Time Point (Months) | mg/ Patch | % Label Claim |
| Initial | 1.06 | 106.0 |
| 3 | 1.02 | 102.0 |
| 6 | 1.02 | 102.0 |
| 9 | 1.00 | 100.0 |
| 12 | 0.99 | 99.0 |
| 18 | 0.98 | 98.0 |
| 24 | 0.97 | 97.0 |

TABLE O -

| Epinephrine Data at 25°C/60% RH in mg/patch and the Percent Label Claim | | |
| --- | --- | --- |
| Time Point (Months) | mg/ Patch | % Label Claim |
| Initial | 1.02 | 102.0 |
| 3 | 1.01 | 101.0 |
| 6 | 1.01 | 101.0 |
| 9 | 0.99 | 99.0 |
| 12 | 0.98 | 98.0 |
| 18 | 0.98 | 98.0 |
| 24 | 0.96 | 96.0 |

TABLE P -

| Epinephrine Data at 25°C/60% RH in mg/patch and the Percent Label Claim | | |
| --- | --- | --- |
| Time Point (Months) | mg/ Patch | % Label Claim |
| Initial | 1.04 | 104.0 |
| 3 | 1.00 | 1000 |
| 6 | 1.00 | 100.0 |
| 9 | 0.99 | 99.0 |
| 12 | 0.98 | 98.0 |
| 18 | 0.98 | 98.0 |
| 24 | 0.96 | 96.0 |

Epinephrine Degradants Assay data for lots 1, 2 and 3 are contained in Tables K, L and M above, respectively. Epinephrine in the patch degrades principally o epinephrine sulfonic acid with minor amounts of adrenolone. At the 24-month time point the epinephrine sulfonic acid is no more than about 43 $\mu$g. This demonstrates that the major route of degradation of epinephrine is actually caused by the major preservative (sodium metabisulfite) used to retard the degradation of epinephrine in the first place. Data on the formation of epinephrine sulfonic acid for lots 1, 2 and 3 show a degradation rate of about 1.6 $\mu$g per month, or about 0.16% per month.

**Lidocaine Hydrochloride Potency and Degradants Assay**

Lidocaine hydrochloride Potency Assay data at 25°C/60% RH for lots 1, 2 and 3 are contained in Tables K, L and M above, respedively. Lidocaine hydrochloride linear regression data together with determination of the 95% lower confidence limit for lots 1, 2 and 3 are contained in Figures 15, 16 and 17, respectively. The equation of the line for each of the three lots is as follows:

Figure 15 - % Label claim for Lot 1 = 101.14-0.208 Time (months)

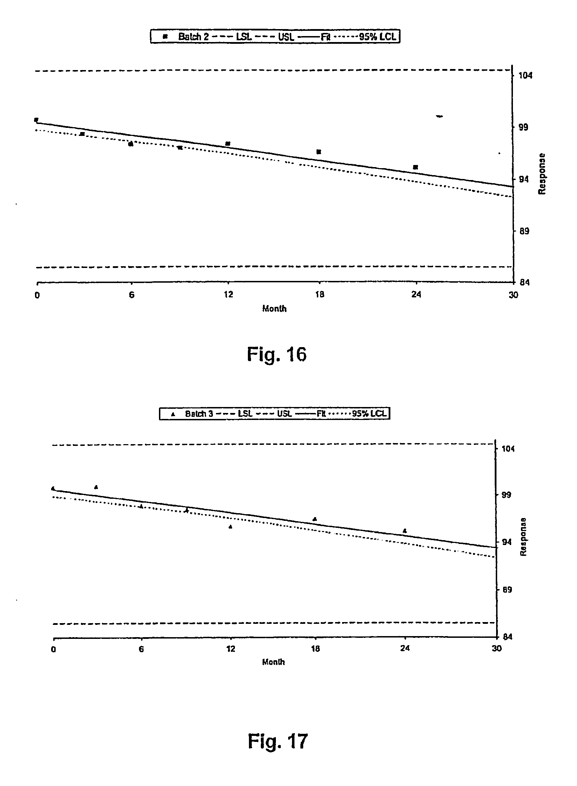

Figure 16 - % Label claim for Lot 2 = 99.526 - 0.208 Time (months)
Figure 17 -% Label claim for Lot 3 = 99.669-0208 Time (months)

For ease of review, the lidocaine hydrochloride potency data used to generate Figures 5, 16 and 17 are included below in Tables Q, R and S, respectively. By projected linear regression, a shelf life of greater than 57 months is obtained.

TABLE Q -

| Lidocaine HCl Data at 25°C/60% RH in mg/patch and Percent Label Claim (Lot 1) | | |
|---|---|---|
| Time Point (Months) | mg/ Patch | % Label Claim |
| Initial | 102.3 | 107.58 |
| 3 | 100.7 | 106.00 |
| 6 | 100.7 | 106.00 |
| 9 | 99.2 | 104.42 |
| 12 | 96.6 | 101.68 |
| 18 | 97.7 | 102.84 |
| 24 | 95.8 | 100.84 |

TABLE R -

| Lidocaine HCl Data at 25°C/60% RH in mg/patch and Percent Label claim (Lot 2) | | |
|---|---|---|
| Time Point (Months) | mg/ Patch | % Label Claim |
| Initial | 99.8 | 105.05 |
| 3 | 98.4 | 103.58 |
| 6 | 97.4 | 102.53 |
| 9 | 97.0 | 102.11 |
| 12 | 97.4 | 102.53 |
| 18 | 96.6 | 101.68 |
| 24 | 95.1 | 100.11 |

TABLE S -

| Lidocaine HCl at 25°C/60% RH Data in mg/patch and Percent Label Claim (Lot 3) | | |
|---|---|---|
| Time Point (Months) | mg/ Patch | % Label Claim |
| Initial | 99.9 | 105.16 |
| 3 | 100.0 | 106.26 |
| 6 | 97.9 | 103.05 |
| 9 | 97.5 | 102.63 |
| 12 | 95.7 | 100.74 |

TABLE S -   (continued)

| Lidocaine HCl at 25°C/60% RH Data in mg/patch and Percent Label Claim (Lot 3) | | |
| --- | --- | --- |
| Time Point (Months) | mg/ Patch | % Label Claim |
| 18 | 96.5 | 101.58 |
| 24 | 95.2 | 100.21 |

A negative slope is associated with the linear regression line for lidocaine hydrochloride with all three lots. The negative slope is not indicative of instability but is indicative of back transfer of the active ingredient from the anode hydrogel reservoir to the transfer pad demonstrated by full material balance including the non-woven at time greater than zero.

Lidocaine hydrochloride Degradants Assay data for lots 1, 2 and 3 are contained in Tables K, L and M, above, respectively. Lidocaine hydrochloride is a stable API. There is no evidence of degradation of lidocaine hydrochloride in the patch. The most likely degradation product of lidocaine hydrochloride, 2,6 dimethylanitine, is not present.

### Preservative Assay/Microbial Limits

The Preservative Assay and Microbial Limits tests for lots 1, 2 and 3 are contained in Tables K, L and M, above, respectively. All results at the initial and 24-month time point for the anode reservoirs are within specification and indicate that the iontophoretic patch is adequately preserved.

### Gel Integrity

The integrity of the anode and cathode hydrogels is assured through the determination of pH, Probe Tack and Apparent Compressive Modulus. The data at 25°C/60% RH for lots 1, 2 and 3 are contained in Tables K, L and M, above, respectively. All tests are within specifications at all time points. The gel remains tacky and the pH remains within the suitable specification for application to the skin.

### Patch Integrity - Physical and Electrochemical

The Probe Tack test of the peripheral adhesive assures the patch remains in contact with the skin. The data at 25°C/60% RH for lots 1, 2 and 3 are contained in Tables K, L and M, above, respectively. The values are within specifications at all time points. The electrochemical tests indicate the conductive traces are remaining intact and that the integrity of the electrodes is not being compromised.

### Pouch Integrity

The opening force and burst strength assure the integrity of the foil/foil pouch (container closure). The data at 25°C/60% RH for lots 1, and 3 are contained in Tables K, L and M, above, respectively. The values are within specifications at all time points.

In sum, the totality of the long-term stability data at 25°C/60% RH for the stability study on the iontophoretic patch are within proposed limits. The stability lots remain within limits for the proposed 24-month shelf life of the product and the least stable entity in the product, epinephrine, has a projected stability to 26 months with a 95% confidence interval. Tests for the actives and degradants of the actives in the anode reservoir, tests for the preservative and microbiological integrity, tests for anode and cathode gel integrity, tests for patch integrity and tests for pouch integrity indicate that the system continues to function as designed.

### C. Stability Data 30°C/60% RH

Intermediate storage stability data on lots 1, 2 and 3 stored at 30°C/60% RH also were collected as for the 5°C and 25°C, but at three month intervals for up to 12 months. The data at the intermediate storage were gathered with the knowledge from previous stability studies that significant change in the product (particularly epinephrine potency) would occur under accelerated storage conditions. With the intermediate storage condition, all data are within the proposed specifications at all time points through 12 months. The data indicate decreased, but acceptable stability of epinephrine at the higher temperature including significant change in the epinephrine potency over the 12-month period.

### D. Stability Data 40°C/75% RH

Accelerated storage stability data on lots 1, 2 and 3 stored at 40°C/75% RH also were collected as for the 5°C and 25°C, but at 1.5 month intervals for up to 6 months. The data at the accelerated storage were gathered with the knowledge from previous stability studies that significant change in the product (particularly epinephrine potency) does occur under accelerated storage conditions. However, with the accelerated storage condition, all data were within the proposed specifications at all time points through six months. Although the data indicate significant change in epinephrine potency at 40°C, the epinephrine potency and degradants remain within proposed specifications over the six-month storage period. The data at 30°C and 40°C are used to project long-term stability at room temperature and are intended to account for short-term excursions over 25°C. At these elevated temperatures, the system components show no extraordinary degradation.

### Example 4 - Reaction of Epinephrine with Sodium Metabisulfite

Sodium metabisulfite is added to the anode formulation in a protective role for the epinephrine to prevent or slow down the react of the epinephrine with oxygen and limit the formation of the two epinephrine oxidation products in the system. However, excessive amounts of sodium metabisulfite are not desirable.
In typical commercial multi-use stoppered glass vial systems for dispensing of epinephrine-containing drug solution, oxygen is continuously introduced into the containers and the effectiveness of the sodium metabisulfite eventually can be reduced to a negligible level through the reintroduction of atmospheric oxygen with each dosage removal. The sodium metabisulfite may be totally consumed in a reaction with oxygen introduced as syringe samples are removed and the removed solution is replaced with atmospheric oxygen according to the following:

$$H_2O + O_2 + Na_2O_5S_2 \Rightarrow Na_2SO_4 + H_2SO_4$$

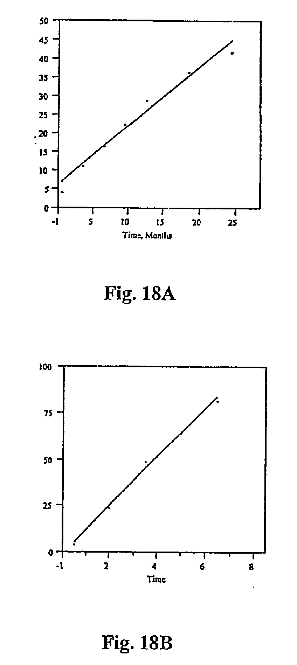

In solution products, once the sodium metabisulfite is consumed, oxidation of epinephrine to adrenalone and adrenochrome becomes the major mode of decomposition of epinephrine. However, due to the design of the packaged iontophoretic device described in the examples above, sodium metabisulfite in excess of amounts needed to scavenge all oxygen present in the hermetically sealed package at the time of packaging is not fully "consumed" during the life of the product, therefore offering continual protection to the epinephrine and extending the shelf life of the products. The described iontophoretic device is a single use product. When the product is initially packaged, the pouch contains up to about 0.5% oxygen and has a headspace of less than 24cc. A larger quantity of sodium metabisulfite was added to cover manufacturing losses and the content of oxygen in the package. The sodium metabisulfite in the anode solution reacts with the oxygen in the closed system, eventually decreasing the overall concentration of oxygen in the closed system to zero. Analysis of the oxygen content in the pouch with time has shown the initial content increase as oxygen is released from under the internal device cover into the patch and then this oxygen content decreases to about zero (0.00%) by the end of about 30 days. The decrease in sodium metabisulfite overtime has been demonstrated by ion chromatographic analysis of the anode hydrogel material for sodium metabisulfite content.
The rate of reaction of the sodium metabisulfite with the oxygen is muca faster than the rate of reaction of oxygen with epinephrine. This mechanism stabilizes the epinephrine by protecting it from the attack by oxygen. This is demonstrated by lack of formation of significant quantities of adrenalone or measurable quantities of adrenochrome in the anode hydrogel during the life of the product. However, epinephrine in the anode hydrogel will form an adduct with the sodium metabisulfite, thereby contributing to the degradation of the epinephrine even in the absence of oxygen. The addition product, epinephrine sulfonic acid, is the product of the reaction of sodium metabisulfite with the hydroxyl group on the amine side chain of epinephrine. The iontophoretic patch is packaged in a hermetically sealed pouch that prevents the reintroduction of oxygen. Once the oxygen content in the pouch reaches zero, the degradation of epinephrine by oxidation is eliminated and the potential for decomposition of the epinephrine shifts to addition product formation.
The rate of formation of epinephrine sulfonic acid is linear when the product is manufactured with an anode formulation containing 0.5 mg/patch of sodium metabisulfite (Figures 18A and 18B). After about two weeks, the typical time of product release, the sodium metabisulfite level already has dropped to about 0.4 - 0.38 mg/patch, illustrating that the sodium metabisulfite is "working" at protecting the epinephrine during the manufacturing process. The protection is further substantiated by the fact that adrenalone and adrenochrome are not formed in the anode hydrogel after the anode solution is applied.

**Example 5 - Passive transdermal patches containing epinephrine**

The data presented above is in reference to a complex iontophoretic system in which shelf stability of the electrode assembly is realized even though the epinephrine-containing reservoir is maintained in contact with a silver/silver chloride electrode. The teachings as to this iontophoresis electrode are fully applicable to passive transdermal devices in which no electrode is present. Such a passive device would be as stable, or more stable than the electrode assemblies described above. A typical passive device would include an epinephrine-containing hydrogel reservoir attached to a backing and would be packaged as is described above. A passive transdermal patch may be assembled and loaded in any manner described above in reference to an electrode assembly, but in a single-reservoir system with no electrodes because no counterelectrode is needed in a passive system.

In addition to the experiments described Examples I through 5, other significant stability studies were conducted at 25°C and followed over time. In one experiment, the patch was tested with no loading absorbent (loaded according to Example 2, above), and passed at 24 months at 25°C. In another, the patch was loaded with excess sodium metabisulfite and failed in less than three months, showing the adverse effect of too much of the preservative used to "protect" the unstable active epinephrine.

The data at 25°C for the patch system support an extended stability of a transdermal hydrogel patch with both lidocaine and epinephrine, with lidocaine alone or with epinephrine alone; in electrotransport reservoir electrodes, passive patches and liquid gels. Because epinephrine is the least stable drug in the studied devices and it is preserved over 24 months at room temperature, these systems are expected to be stable with local anesthetics other than lidocaine, such as without limitation pivocaine and procaine.

Whereas particular embodiments of the invention have been described herein for the purpose of illustrating the invention and not for the purpose of limiting the same, it will be appreciated by those of ordinary skill in the art that numerous variations of the details, materials and arrangement of parts may be made within the principle and scope of the invention without departing from the invention as described in the appended claims.

**Claims**

1. A hermetically-sealed electrode assembly for an electrically assisted drug delivery device, comprising an anode assembly comprising a first electrode and a donor hydrogel containing epinephrine in electrical contact with the first electrode, wherein the hermetically-sealed anode assembly is physically, chemically, electronically, electrochemically or microbiologically stable for at least 12 months at 25°C.

2. The electrode assembly of claim 1, wherein the epinephrine in the donor hydrogel degrades to no more than 90% of original levels for at least 24 months at 25°C.

3. The electrode assembly of any preceding claim, wherein the anode assembly is physically stable for at least 24 months at 25°C.

4. The electrode assembly of any preceding claim, wherein the anode assembly is chemically stable for at least 24 months at 25°C.

5. The electrode assembly of any preceding claim, wherein the anode assembly is electrochemically stable for at least 10 months at 25°C.

6. The electrode assembly of any preceding claim, wherein the anode assembly is physically, chemically, electronically, electrochemically and microbiologically stable for at least six months at 40°C.

7. The electrode assembly of any preceding claim, wherein the anode assembly is physically, chemically, electronically, electrochemically and microbiologically stable for at least 12 months at 30°C.

8. The electrode assembly of any preceding claim, wherein the donor hydrogel comprises a local anesthetic.

9. The electrode assembly of claim 8, wherein the local anesthetic is lidocaine.

10. The electrode assembly of any preceding claim, further comprising a cathode assembly comprising a second electrode and a return hydrogel in electrical contact with the second electrode, wherein the anode and cathode electrodes are attached to a backing.

11. The electrode assembly of claim 10, further comprising an electrically conductive anode trace attached to the backing and electrically connected to the first electrode and an electrically conductive cathode trace attached to the backing and electrically connected to the second electrode.

12. The electrode assembly of claim 11, wherein the anode and cathode traces are coated with a dielectric material.

13. The electrode assembly of claim 11, wherein the first and second electrodes and the anode and cathode traces are printed with silver/silver chloride-containing ink.

14. The electrode assembly of claim 10, wherein the first and second electrodes are silver/silver chloride electrodes.

15. The electrode assembly of any preceding claim, wherein the epinephrine in the donor hydrogel degrades to no more than about 95% wt. of original levels in 24 months at 25°C.

16. The electrode assembly of any preceding claim, the epinephrine in the donor hydrogel degrading to no more than about 94% wt. of original levels in 18 months at 25°C.

17. The electrode assembly of any preceding claim, wherein the hydrogel comprises polyvinyl pyrrolidone.

18. The electrode assembly of claim 17, wherein the hydrogel is about 17% wt. polyvinyl pyrrolidone.

19. The electrode assembly of any preceding claim, wherein the first electrode is a silver or a silver/silver chloride electrode.

20. The electrode assembly of any preceding claim, wherein the donor hydrogel contains an amount of sodium metabisulfite equal to or slightly greater than a minimal amount of sodium metabisulfite needed to scavenge oxygen in the packaged donor hydrogel for at least 24 months.

21. The electrode assembly of claim 20, wherein the donor hydrogel contains less than about 110% of the amount of sodium metabisulfite equal to a minimal amount of sodium metabisulfite needed to scavenge oxygen in the packaged donor hydrogel for at least 24 months.

22. The electrode assembly of claim 20, wherein the donor hydrogel contains about 101% of the amount of sodium metabisulfite equal to a minimal amount of sodium metabisulfite needed to scavenge oxygen in the packaged donor hydrogel for at least 24 months.

23. The electrode assembly of any preceding claim, wherein the donor hydrogel comprises from about 2% wt. to about 12% wt. lidocaine and from about 0.001% wt. to about 0.3% wt epinephrine.

24. The electrode assembly of claim 23, wherein the donor hydrogel has a volume of about 1ml and comprises about 100 mg lidocaine HCl and about 1.05 mg epinephrine bitartrate.

25. The electrode assembly of claim 24, wherein the donor hydrogel is about 40 mil ± 5 mil thick.

26. The electrode assembly of any preceding claim, wherein the donor hydrogel comprises lidocaine HCl and epinephrine bitartrate in about a 70-125:1 mass ratio.

27. The electrode assembly of any preceding claim, the donor hydrogel further contains lidocaine and one or more of parabens, sodium metabisulfite, a chelating agent, citric acid, glycerin and sodium chloride.

28. The electrode assembly of any preceding claim, wherein the donor electrode is prepared by contacting a solution containing the lidocaine and the epinephrine with an unloaded hydrogel containing from about 0.001% wt. to about 1.0% sodium chloride.

29. The electrode assembly of any preceding claim, packaged under an inert atmosphere.

30. The electrode assembly of claim 29, wherein the inert gas is nitrogen.

31. An electrode assembly for an electrically assisted drug delivery device packaged in an inert atmosphere in a hermetically-sealed container, the electrode assembly comprising:

    (a) a backing;
    (b) a first silver/silver chloride electrode attached to the backing and a donor hydrogel comprising polyvinyl pyrrolidone and containing lidocaine and epinephrine in electrical contact with the first electrode;
    (c) a second silver/silver chloride electrode attached to the backing and a return hydrogel in electrical contact with the second electrode;
    (d) an electrically conductive silver or silver/silver chloride anode trace attached to the backing and in electrical contact with the first electrode;
    (e) an electrically conductive silver or silver/silver chloride cathode trace attached to the backing and in electrical contact with the second electrode; and
    (f) a dielectric layer coating at least a portion of the anode and cathode traces,

    wherein the donor hydrogel contains an amount of sodium metabisulfite equal to or slightly greater than a minimal amount of sodium metabisulfite needed to scavenge oxygen in the donor hydrogel for at least 10 months and an amount of salt sufficient to prevent electrode corrosion during or after loading of the hydrogel reservoir,
    wherein the first and second electrodes and the anode and cathode traces are printed, and
    wherein the anode assembly is stable for at least 10 months at 25°C.

32. A method for preparing a shelf-stable electrode assembly for electrically-assisted delivery of a local anesthetic and vasoconstrictor to a patient, the electrode assembly comprising an unloaded hydrogel reservoir in electrical contact with a silver-silver chloride electrode, the unloaded hydrogel reservoir containing an amount of salt sufficient to prevent electrode corrosion during or after loading of the hydrogel reservoir; the method comprising:

    (a) loading the unloaded hydrogel reservoir with a loading solution containing lidocaine and epinephrine; and
    (b) packaging the assembly in an inert atmosphere in a hermetically-sealed container.

33. The method of claim 32, wherein, prior to the loading, the loading solution is absorbed into an absorbent pad attached to a releasable liner configured to cover the hydrogel reservoir, and the releasable liner is attached to the electrode assembly with the absorbent pad contacting the hydrogel reservoir, thereby contacting the loading solution with the hydrogel.

34. An electrode assembly for an electrically assisted drug delivery device, comprising a first electrode and a donor hydrogel comprising lidocaine and epinephrine in electrical contact with the first electrode, wherein the electrode assembly is electrically stable, physically stable and chemically stable for at least 12 months at 25°C when the anode assembly is hermetically-sealed.

35. A packaged electrode assembly for an electrically assisted drug delivery device, comprising a hermetically-sealed container and an electrode assembly sealed within the container, the electrode assembly comprising a first electrode and a donor polyvinyl pyrrolidone comprising hydrogel comprising lidocaine and epinephrine in electrical contact with a silver/silver chloride first electrode, wherein the packaged electrode assembly is electrically stable, physically stable and chemically stable for at least 12 months at 25°C when the anode assembly is hermetically sealed.

36. The packaged electrode assembly of claim 35, wherein the donor hydrogel contains an amount of sodium metabisulfite equal to or slightly greater than a minimal amount of sodium metabisulfite needed to scavenge oxygen in the packaged donor hydrogel for at least 24 months.

37. A packaged electrode assembly for an electrically assisted drug delivery device, comprising a hermetically-sealed electrode comprising an anode assembly, the anode assembly comprising a first electrode and a donor hydrogel containing a vasoconstrictor, an anesthetic and pharmaceutically acceptable excipients in electrical contact with the first electrode, wherein the electrode assembly is physically, chemically, electronically, electrochemically or microbiologically stable for at least 12 months at 25°C.

38. The packaged electrode assembly of claim 37, wherein the vasoconstrictor is one of epinephrine and phenylephrine.

**39.** The packaged electrode assembly of claim 37, wherein the anesthetic is selected tom the group consisting of amide type anesthetics, ester type anesthetics, bupivacaine, butanilicaine, carticaine, cinchocaine/dibucaine, clibucaine, ethyl parapiperidino acetylaminobenzoate, etidocaine, lidocaine, mepivicaine, oxethazaine, prilocaine, ropivicaine, tolycaine, trimecaine, vadocaine, amylocaine, cocaine, propanocaine, esters of metaaminobenzoic acid, clormecaine, proxymetacaine, esters of paraaminobenzoic acid, amethocaine, benzocaine, butacaine, butoxycaine, butyl aminobenzoate, chloroprocaine, oxybuprocaine, parethoxycaine, procaine, propoxycaine, tricaine, bucricaine, dimethisoquin, diperodon, dyclocaine, ethyl chloride, ketocaine, myrtecaine, octacaine, pramoxine and propipocaine.

**40.** The packaged electrode assembly of claim 37, wherein the anesthetic is one of bupivacaine, butacaine, chloroprocaine, cinchocaine, etidocaine, mepivacaine, prilocaine, procaine, ropivacaine and tetracaine.

**41.** The packaged electrode assembly of claim 37, wherein the anesthetic is one of bupivacaine, etidocaine, mepivacaine, ropivicaine and prilocaine.

**42.** The packaged electrode assembly of claim 37, wherein the anesthetic is lidocaine.

**43.** The packaged electrode assembly of claim 42, wherein donor hydrogel comprises between about 2% wt. to about 12% wt. lidocaine.

**44.** The packaged electrode assembly of claim 42, wherein donor hydrogel comprises between about 5% wt. to about 12% wt. lidocaine.

**45.** The packaged electrode assembly of claim 42, wherein donor hydrogel comprises between about 8% wt. to about 12% wt. lidocaine.

**46.** The packaged electrode assembly of any of claims 37 to 45, wherein the electrode assembly is physically, chemically, electronically, electrochemically or microbiologically stable for at least 18 months at 25°C.

**47.** The packaged electrode assembly of any of claims 37 to 45, wherein the electrode assembly is physically, chemically, electronically, electrochemically or microbiologically stable for at least 10 months at 25°C.

**48.** The packaged electrode assembly of any of claims 37 to 45, wherein the electrode assembly is physically, chemically, electronically, electrochemically or microbiologically stable for at least 36 months at 25°C.

**49.** The packaged electrode assembly of any of claims 37 to 45, wherein the electrode assembly is physically, chemically, electronically, electrochemically or microbiologically stable for at least 48 months at 25°C.

**50.** The packaged electrode assembly of any of claims 37 to 49, wherein the donor hydrogel comprises from about 0.001% wt. to about 0,3% wt. epinephrine.

**51.** The packaged electrode assembly of any of claims 37 to 49, wherein the donor hydrogel comprises from about 0.01% wt. to about 0.3% wt. epinephrine.

**52.** The packaged electrode assembly of any of claims 37 to 49, wherein the donor hydrogel comprises from about 0.075% wt. to about 0.125% wt. epinephrine.

**53.** The packaged electrode assembly of any of claims 37 to 52, wherein the donor hydrogel comprises from about 0.005% wt. to about 0.1% wt. sodium metabisulfite.

**54.** The packaged electrode assembly of any of claims 37 to 52, wherein the donor hydrogel comprises from about 0.025% wt. to about 0.075% wt. sodium metabisulfite.

**55.** The packaged electrode assembly of any of claims 37 to 54, wherein the donor hydrogel comprises from about 0.01% wt. to about 0.1% wt. sodium chloride.

**56.** The packaged electrode assembly of any of claims 37 to 55, wherein the donor hydrogel comprises polyvinylpyrrolidone.

**57.** The packaged electrode assembly of any of claims 37 to 56, wherein the first electrode is a silver or silver/silver chloride electrode.

**58.** The packaged electrode assembly of any of claims 37 to 56, wherein the first electrode is a printed silver or silver/ silver chloride electrode.

**59.** The packaged electrode assembly of any of claims 37 to 58, wherein the electrode is packaged in an inert atmosphere.

**60.** The packaged electrode assembly of claim 59, wherein the inert atmosphere comprises nitrogen.

**61.** The packaged electrode assembly of claim 37, wherein the donor hydrogel is a polyvinylpyrrolidone hydrogel comprising lidocaine, epinephrine, sodium metabisulfite, citric acid, sodium chloride, a chelating agent, parabens and glycerin and the electrode is packaged in an inert atmosphere.

**62.** A packaged electrode assembly for an electrically assisted drug delivery device, comprising a hermetically-sealed electrode packaged in an inert atmosphere, the electrode comprising an anode assembly, the anode assembly comprising a first electrode and a polyvinylpyrrolidone donor hydrogel in electrical contact with the first electrode, the hydrogel containing lidocaine, epinephrine, sodium metabisulfite, citric acid, sodium chloride, parabens and glycerin, wherein the electrode assembly is physically, chemically, electronically, electrochemically or microbiologically stable for at least 12 months at 25°C.

**63.** A transdermal patch comprising epinephrine that is stable at 25°C for at least about 24 months.

**64.** An electrotransport reservoir comprising epinephrine that is stable at 25°C for at least about 24 months.

**65.** A packaged transdermal drug delivery device, comprising a hermetically-sealed polyvinylpyrrolidone hydrogel packaged in an inert atmosphere, the hydrogel containing lidocaine, epinephrine, sodium metabisulfite, citric acid, sodium chloride, a chelating agent, parabens and glycerin, wherein the electrode assembly is physically, chemically or microbiologically stable for at least 12 months at 25°C.

**66.** The packaged electrode assembly of claim 65, wherein donor hydrogel comprises between about 2% wt. to about 12% wt. lidocaine.

**67.** The packaged electrode assembly of claim 65, wherein donor hydrogel comprises between about 5% wt. to about 12% wt. lidocaine.

**68.** The packaged electrode assembly of claim 65, wherein donor hydrogel comprises between about 8% wt. to about 12% wt. lidocaine.

**69.** The packaged electrode assembly of any of claims 65 to 68, wherein the electrode assembly is physically, chemically, electrochemically, electrically or microbiologically stable for at least 18 months at 25°C.

**70.** The packaged electrode assembly of any of claims 65 to 68, wherein the electrode assembly is physically, chemically, electrochemically, electrically or microbiologically stable for at least 24 months at 25°C.

**71.** The packaged electrode assembly of any of claims 65 to 68, wherein the electrode assembly is physically, chemically, electrochemically, electrically or microbiologically stable for at least 36 months at 25°C.

**72.** The packaged electrode assembly of any of claims 65 to 68, wherein the electrode assembly is physically, chemically, electrochemically, electrically or microbiologically stable for at least 48 months at 25°C.

**73.** The packaged electrode assembly of any of claims 65 to 72, wherein the donor hydrogel comprises from about 0.001% wt. to about 0.3% wt. epinephrine.

**74.** The packaged electrode assembly of any of claims 65 to 72, wherein the donor hydrogel comprises from about 0.01% wt. to about 0.3% wt. epinephrine.

**75.** The packaged electrode assembly of any of claims 65 to 72, wherein the donor hydrogel comprises from about 0.075% wt. to about 0.125% wt. epinephrine.

**76.** The packaged electrode assembly of any of claims 65 to 75, wherein the donor hydrogel comprises from about 0.005% wt. to about 0.1% wt sodium metabisulfite.

**77.** The packaged electrode assembly of any of claims 65 to 75, wherein the donor hydrogel comprises from about 0.025% wt. to about 0.075% wt. sodium metabisulfite.

**78.** The packaged electrode assembly of any of claims 65 to 77, wherein the donor hydrogel comprises from about 0.01% wt. to about 0.1% wt. sodium chloride.

FIG. 1  (Prior Art)

FIG. 4

FIG. 3

FIG. 2

FIG. 5A

FIG. 5B

FIG. 5C

EP 1 586 347 A1

FIG. 6B

FIG. 6C

FIG. 10

FIG. 6

FIG. 7

FIG. 7A

FIG. 8

FIG. 9

FIG. 11

Fig. 12

Fig. 13

Fig. 14

Fig. 15

Fig. 16

Fig. 17

Fig. 18A

Fig. 18B

**European Patent Office**

## EUROPEAN SEARCH REPORT

Application Number

EP 05 25 2156

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00/74772 A (BECTON, DICKINSON AND COMPANY) 14 December 2000 (2000-12-14) * page 24, line 10 - line 14 * * page 17, line 5 - line 13 * | 1-78 | A61N1/30 |
| D,Y | WO 01/91848 A (BECTON, DICKINSON AND COMPANY; BERNHARD, MICHAEL, I; EWALL, RALPH; KAR) 6 December 2001 (2001-12-06) * page 7, line 10 - line 13 * * page 8, line 14 - page 9, line 16 * | 1-78 | |
| D,Y | WO 98/20869 A (ALZA CORPORATION) 22 May 1998 (1998-05-22) * page 21, line 3 - line 10 * | 1-78 | |
| A | EP 0 368 408 A (THE PROCTER & GAMBLE COMPANY) 16 May 1990 (1990-05-16) * abstract * | 1-78 | |
| A | WO 96/10398 A (BECTON DICKINSON AND COMPANY) 11 April 1996 (1996-04-11) * page 4, line 8 - line 18 * | 1-78 | TECHNICAL FIELDS SEARCHED (Int.Cl.7) A61K A61N |
| D,A | US 6 635 045 B2 (KEUSCH PRESTON ET AL) 21 October 2003 (2003-10-21) * column 2, line 10 - column 3, line 15 * | 1-78 | |
| D,A | US 6 629 968 B1 (JAIN UDAY K ET AL) 7 October 2003 (2003-10-07) * column 3, line 49 - column 6, line 46 * | 1-78 | |

The present search report has been drawn up for all claims

| Place of search | Date of completion of the search | Examiner |
|---|---|---|
| The Hague | 5 August 2005 | Petter, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 05 25 2156

This annex lists the patent family members relating to the patent documents cited in  the above-mentioned European search report.
The members are as contained in the European Patent Office EDP file on
The European Patent Office is in no way liable for these particulars which are merely  given for the purpose of information.

05-08-2005

| Patent document<br>cited in search report | | Publication<br>date | Patent family<br>member(s) | | Publication<br>date |
|---|---|---|---|---|---|
| WO 0074772 | A | 14-12-2000 | CA | 2364414 A1 | 06-06-2003 |
| | | | US | 6377847 B1 | 23-04-2002 |
| | | | EP | 1207936 A1 | 29-05-2002 |
| | | | JP | 2003501165 T | 14-01-2003 |
| | | | WO | 0074772 A1 | 14-12-2000 |
| | | | US | 2003055405 A1 | 20-03-2003 |
| | | | US | 2005159698 A1 | 21-07-2005 |
| | | | US | 2005131338 A1 | 16-06-2005 |
| | | | US | 2005159699 A1 | 21-07-2005 |
| | | | US | 2005159700 A1 | 21-07-2005 |
| WO 0191848 | A | 06-12-2001 | US | 6496727 B1 | 17-12-2002 |
| | | | AT | 278436 T | 15-10-2004 |
| | | | AU | 7501901 A | 11-12-2001 |
| | | | BR | 0111367 A | 20-05-2003 |
| | | | CA | 2410675 A1 | 06-12-2001 |
| | | | CN | 1481264 A | 10-03-2004 |
| | | | DE | 60106235 D1 | 11-11-2004 |
| | | | DK | 1284785 T3 | 07-02-2005 |
| | | | EP | 1284785 A2 | 26-02-2003 |
| | | | EP | 1473056 A1 | 03-11-2004 |
| | | | ES | 2230327 T3 | 01-05-2005 |
| | | | JP | 2003534107 T | 18-11-2003 |
| | | | MX | PA02011849 A | 14-05-2003 |
| | | | WO | 0191848 A2 | 06-12-2001 |
| | | | US | 2003065304 A1 | 03-04-2003 |
| WO 9820869 | A | 22-05-1998 | AT | 238046 T | 15-05-2003 |
| | | | AU | 721114 B2 | 22-06-2000 |
| | | | AU | 5166398 A | 03-06-1998 |
| | | | CA | 2271944 A1 | 22-05-1998 |
| | | | CN | 1237907 A ,C | 08-12-1999 |
| | | | DE | 69721293 D1 | 28-05-2003 |
| | | | DE | 69721293 T2 | 29-01-2004 |
| | | | DK | 941085 T3 | 11-08-2003 |
| | | | EP | 0941085 A2 | 15-09-1999 |
| | | | ES | 2194222 T3 | 16-11-2003 |
| | | | JP | 2001505197 T | 17-04-2001 |
| | | | KR | 2000053270 A | 25-08-2000 |
| | | | PT | 941085 T | 29-08-2003 |
| | | | WO | 9820869 A2 | 22-05-1998 |
| EP 0368408 | A | 16-05-1990 | US | 5008110 A | 16-04-1991 |
| | | | CA | 2002298 A1 | 10-05-1990 |
| | | | EP | 0368408 A2 | 16-05-1990 |
| | | | JP | 2237915 A | 20-09-1990 |

EPO FORM P0459

For more details about this annex : see Official Journal of the  European Patent Office, No. 12/82

**ANNEX TO THE EUROPEAN SEARCH REPORT
ON EUROPEAN PATENT APPLICATION NO.** EP 05 25 2156

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2005

| Patent document cited in search report | | Publication date | Patent family member(s) | | Publication date |
|---|---|---|---|---|---|
| EP 0368408 | A | | JP 2957209 B2 | | 04-10-1999 |
| WO 9610398 | A | 11-04-1996 | AU | 3761495 A | 26-04-1996 |
| | | | CA | 2201354 A1 | 11-04-1996 |
| | | | DE | 69506122 D1 | 24-12-1998 |
| | | | DK | 783301 T3 | 02-08-1999 |
| | | | EP | 0783301 A1 | 16-07-1997 |
| | | | JP | 10509694 T | 22-09-1998 |
| | | | WO | 9610398 A1 | 11-04-1996 |
| | | | US | 5682726 A | 04-11-1997 |
| US 6635045 | B2 | 23-05-2002 | US | 6629968 B1 | 07-10-2003 |
| | | | US | 2002062102 A1 | 23-05-2002 |
| | | | AU | 7310401 A | 14-01-2002 |
| | | | CA | 2413624 A1 | 10-01-2002 |
| | | | EP | 1294439 A2 | 26-03-2003 |
| | | | JP | 2004501727 T | 22-01-2004 |
| | | | WO | 0202182 A2 | 10-01-2002 |
| US 6629968 | B1 | 07-10-2003 | AU | 7310401 A | 14-01-2002 |
| | | | CA | 2413624 A1 | 10-01-2002 |
| | | | EP | 1294439 A2 | 26-03-2003 |
| | | | JP | 2004501727 T | 22-01-2004 |
| | | | WO | 0202182 A2 | 10-01-2002 |
| | | | US | 2002062102 A1 | 23-05-2002 |

EPO FORM P0459

For more details about this annex : see Official Journal of the European Patent Office, No. 12/82